# EUROPEAN PATENT APPLICATION

(11) **EP 4 803 518 A1**
(43) Date of publication of application: **09.09.2026**
(21) Application number: 24885788.0
(22) Date of filing: 30.10.2024
(51) Int. Cl.: C07D 451/14, B01J 31/02, C07B 61/00, C07C 45/29, C07C 47/228, C07C 49/04, C07C 49/76, C07C 49/175, C07C 49/385, C07C 49/657, C07C 201/12, C07C 205/44, C07F 7/18

(54) **TETRAZENE COMPOUND, TETRAZENE RADICAL SALT COMPOUND, TETRAZENE-TYPE ALCOHOL OXIDATION CATALYST, TETRAZENE RADICAL SALT-TYPE ALCOHOL OXIDATION CATALYST, AND ALCOHOL OXIDATION METHOD USING SAME**

(30) Priority: 30.10.2023 JP 2023185949
(71) Applicant: Tohoku University, Sendai-shi, Miyagi 980-8577 (JP)
(72) Inventor: SASANO Yusuke, Sendai-shi, Miyagi 980-8577 (JP); IWABUCHI Yoshiharu, Sendai-shi, Miyagi 980-8577 (JP); OSHIRO Ayari, Sendai-shi, Miyagi 980-8577 (JP); KWON Eunsang, Sendai-shi, Miyagi 980-8577 (JP); SAITO Shu, Sendai-shi, Miyagi 980-8577 (JP)
(74) Representative: Plasseraud IP
(86) International application number: PCT/JP2024/038772
(87) International publication number: WO 2025/095021

(57) **Abstract**

Provided are a novel tetrazene compound, a novel tetrazene radical salt compound, a novel tetrazene-type alcohol oxidation catalyst, a novel tetrazene radical salt-type alcohol oxidation catalyst, and a alcohol oxidation method using them, all of which are different from the above polycyclic N-oxyl compound, are easy to produce, and are suitable for use as a alcohol oxidation catalyst capable of exhibiting sufficiently high catalytic activity even in the oxidation of secondary alcohol. The tetrazene compound has an adamantane skeleton represented by chemical formula (1) below or a bicyclic skeleton represented by chemical formula (2) below.

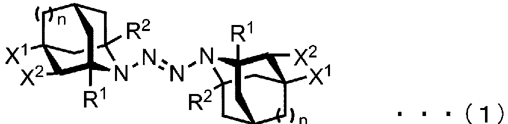

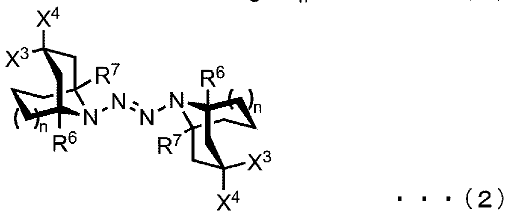

## Description

### Technical Field

The present invention relates to tetrazene compounds, tetrazene radical salt compounds, tetrazene-type alcohol oxidation catalysts, tetrazene radical salt-type alcohol oxidation catalysts, and alcohol oxidation methods using them.

The present application claims priority to Japanese Patent Application No. 2023-185949 filed in Japan on October 30, 2023, and the contents of which are incorporated herein.

### Background Art

An alcohol oxidation reaction for oxidizing an alcohol into a carbonyl compound is one of most fundamental reactions used in organic synthesis of other fine chemicals such as pharmaceuticals, agrochemicals, aroma chemicals, and fine chemical products, and various methods have been developed so far. However, the methods in the related art have problems in terms of safety and environmental impact, such as necessity of heating to a high temperature, necessity of using a toxic or explosive oxidant, and generation of a large amount of waste containing heavy metals.

With such problems as a background, in recent years, 2,2,6,6-tetramethylpiperidine 1-oxyl (hereinafter, referred to as "TEMPO") has attracted attention as a catalyst capable of implementing oxidation of alcohols even on a large scale from the viewpoint of being able to oxidize alcohols under extremely mild conditions from 0 °C to room temperature using various cooxidants without using a highly toxic or hazardous reagent.

Further, recently, the present inventors have reported that polycyclic N-oxyl compounds such as an N-oxyl (nitroxyl radical) compound having an azaadamantane skeleton (2-azaadamantane N-oxyl (hereinafter referred to as "AZADO") and 1-methyl-2-azaadamantane N-oxyl (hereinafter referred to as "1-Me-AZADO")), an N-oxyl compound having an azabicyclo[3.3.1]nonane skeleton (9-azabicyclo[3.3.1]nonane N-oxyl, hereinafter referred to as "ABNO"), and an N-oxyl compound having an azanoradamantane skeleton (9-azanoradamantane N-oxyl, hereinafter referred to as "Nor-AZADO") may have alcohol oxidation catalytic activity higher than that of TEMPO (for example, see PTLs 1 to 3 and NPLs 1 to 4).

### Citation List

### Patent Literature

PTL 1: WO2006/001387
PTL 2: JP2008-212853A
PTL 3: WO2012/008228

### Non Patent Literature

NPL 1: Shibuya, M. et al., J. Am. Chem. Soc., 2006, Vol. 128, No. 26, pp. 8412-8413
NPL 2: Shibuya, M. et al., J. Org. Chem., 2009, Vol. 74, No. 12, pp. 4619-4622
NPL 3: Shibuya, M. et al., Synthesis, 2011, No. 21, pp. 3418-3425
NPL 4: Hayashi, M. et al., Chem. Pharm. Bull., 2011, Vol. 59, No. 12, pp. 1570-1573

### Summary of Invention

### Technical Problem

The invention has been made in view of the problems in the related art, and an object of the invention is to provide new tetrazene compounds, new tetrazene radical salt compounds, new tetrazene-type alcohol oxidation catalysts, and new tetrazene radical salt-type alcohol oxidation catalysts that are each suitably applicable as an alcohol oxidation catalyst capable of exhibiting a sufficiently high catalytic activity even in oxidation of secondary alcohols, that are easy to produce, and that are each different from the above polycyclic N-oxyl compounds (AZADO, 1-Me-AZADO, ABNO, and Nor-AZADO), and alcohol oxidation methods using them.

### Solution to Problem

As a result of intensive studies to achieve the above object, the present inventors have found new tetrazene compounds having adamantane skeletons at both ends of tetrazene, and have found that a sufficiently high alcohol oxidation catalytic activity is exhibited even for bulky secondary alcohols by using the tetrazene compounds as alcohol oxidation catalysts.

To date, there have been no reports on such a tetrazene compound having an adamantane skeleton, and the present inventors were the first to find the tetrazene compounds having adamantane skeletons, the tetrazene radical salt compounds, the tetrazene-type alcohol oxidation catalysts, the tetrazene radical salt-type alcohol oxidation catalysts, and the fact that an alcohol can be oxidized using these catalysts. Further, the present inventors have found that the tetrazene compounds having adamantane skeletons and the tetrazene radical salt compounds can be easily produced in several steps, and have completed the present invention.

The invention includes the following aspects.
[1] A tetrazene compound having an adamantane skeleton represented by the following chemical formula (1) or a bicyclic skeleton represented by the following chemical formula (2). 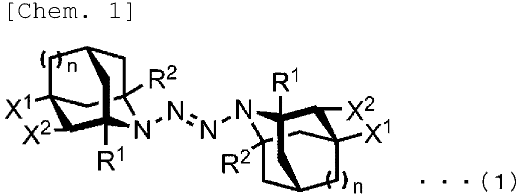 (In the formula (1), n is 0 or 1, R¹ is H, an alkyl group having 1 to 6 carbon atoms that may include a substituent, a phenyl group that may include a substituent, a carboxy group, an amide group, or an alkoxycarbonyl group having 1 to 6 carbon atoms, R² is H, an alkyl group having 1 to 6 carbon atoms that may include a substituent, a carboxy group, an amide group, or an alkoxycarbonyl group having 1 to 6 carbon atoms, X¹ is H, OR³ (R³ is H, an alkyl group having 1 to 6 carbon atoms that may include a substituent, an acyl group, or a silyl group having 3 to 18 carbon atoms), NR⁴R⁵ (R⁴ and R⁵ are each independently H, an alkyl group having 1 to 6 carbon atoms that may include a substituent, an acyl group, an alkoxycarbonyl group, or a group obtained by substituting OH of a sulfo group with a monovalent group, and R⁴ and R⁵ may be bonded to each other to form a ring), a halogen atom, a phenyl group that may include a substituent, or an alkyl group having 1 to 6 carbon atoms that may include a substituent, X² is H, OR³ (R³ is H, an alkyl group having 1 to 6 carbon atoms that may include a substituent, an acyl group, or a silyl group having 3 to 18 carbon atoms), NR⁴R⁵ (R⁴ and R⁵ are each independently H, an alkyl group having 1 to 6 carbon atoms that may include a substituent, an acyl group, an alkoxycarbonyl group, or a group obtained by substituting OH of a sulfo group with a monovalent group, and R⁴ and R⁵ may be bonded to each other to form a ring), or a halogen atom, and either X¹ or X² is H.) 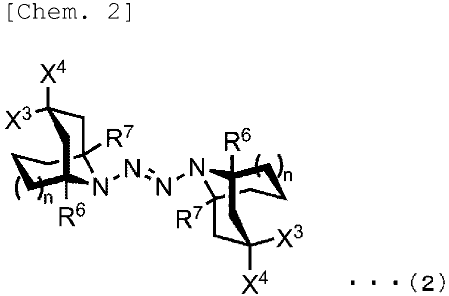 (In the formula (2), n is 0 or 1, R⁶ and R⁷ are each independently H or an alkyl group having 1 to 6 carbon atoms that may include a substituent, X³ and X⁴ are each independently H, an alkyl group having 1 to 6 carbon atoms that may include a substituent, an aromatic group that may include a substituent, OR³ (R³ is H, an alkyl group having 1 to 6 carbon atoms that may include a substituent, an acyl group, or a silyl group having 3 to 18 carbon atoms), NR⁴R⁵ (R⁴ and R⁵ are each independently H, an alkyl group having 1 to 6 carbon atoms that may include a substituent, an acyl group, an alkoxycarbonyl group, or a group obtained by substituting OH of a sulfo group with a monovalent group, and R⁴ and R⁵ may be bonded to each other to form a ring), or a halogen atom, or X³ and X⁴ may form a keto group, an imino group (NR⁸), an oxime group (NOR⁹), or a hydrazone group (NNR¹⁰R¹¹), the R⁸ to R¹¹ are each independently H, an alkyl group having 1 to 6 carbon atoms that may include a substituent, a phenyl group that may include a substituent, an acyl group, an alkoxycarbonyl group, or a group obtained by substituting OH of a sulfo group with a monovalent group, R¹⁰ and R¹¹ may be bonded to each other to form a ring, X³ and X⁴ may be bonded to each other to form a ring, and one of R⁶, R⁷, X³, and X⁴ is not H.)
[2] A tetrazene radical salt compound having an adamantane skeleton represented by the following chemical formula (3) or a bicyclic skeleton represented by the following chemical formula (4). 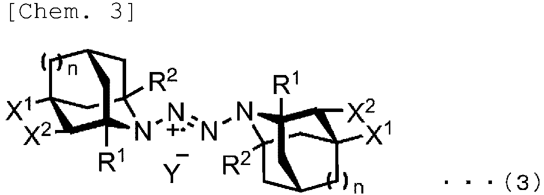 (In the formula (3), n is 0 or 1, R¹ is H, an alkyl group having 1 to 6 carbon atoms that may include a substituent, a phenyl group that may include a substituent, a carboxy group, an amide group, or an alkoxycarbonyl group having 1 to 6 carbon atoms, R² is H, an alkyl group having 1 to 6 carbon atoms that may include a substituent, a carboxy group, an amide group, or an alkoxycarbonyl group having 1 to 6 carbon atoms, X¹ is H, OR³ (R³ is H, an alkyl group having 1 to 6 carbon atoms that may include a substituent, an acyl group, or a silyl group having 3 to 18 carbon atoms), NR⁴R⁵ (R⁴ and R⁵ are each independently H, an alkyl group having 1 to 6 carbon atoms that may include a substituent, an acyl group, an alkoxycarbonyl group, or a group obtained by substituting OH of a sulfo group with a monovalent group, and R⁴ and R⁵ may be bonded to each other to form a ring), a halogen atom, a phenyl group that may include a substituent, or an alkyl group having 1 to 6 carbon atoms that may include a substituent, X² is H, OR³ (R³ is H, an alkyl group having 1 to 6 carbon atoms that may include a substituent, an acyl group, or a silyl group having 3 to 18 carbon atoms), NR⁴R⁵ (R⁴ and R⁵ are each independently H, an alkyl group having 1 to 6 carbon atoms that may include a substituent, an acyl group, an alkoxycarbonyl group, or a group obtained by substituting OH of a sulfo group with a monovalent group, and R⁴ and R⁵ may be bonded to each other to form a ring), or a halogen atom, either X¹ or X² is H, and Y⁻ is a monovalent anion.) 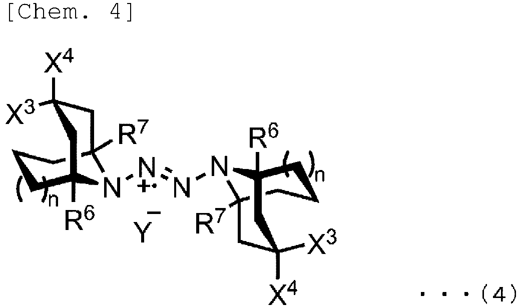 (In the formula (4), n is 0 or 1, R⁶ and R⁷ are each independently H or an alkyl group having 1 to 6 carbon atoms that may include a substituent, X³ and X⁴ are each independently H, an alkyl group having 1 to 6 carbon atoms that may include a substituent, an aromatic group that may include a substituent, OR³ (R³ is H, an alkyl group having 1 to 6 carbon atoms that may include a substituent, an acyl group, or a silyl group having 3 to 18 carbon atoms), NR⁴R⁵ (R⁴ and R⁵ are each independently H, an alkyl group having 1 to 6 carbon atoms that may include a substituent, an acyl group, an alkoxycarbonyl group, or a group obtained by substituting OH of a sulfo group with a monovalent group, and R⁴ and R⁵ may be bonded to each other to form a ring), or a halogen atom, or X³ and X⁴ may form a keto group, an imino group (NR⁸), an oxime group (NOR⁹), or a hydrazone group (NNR¹⁰R¹¹), the R⁸ to R¹¹ are each independently H, an alkyl group having 1 to 6 carbon atoms that may include a substituent, a phenyl group that may include a substituent, an acyl group, an alkoxycarbonyl group, or a group obtained by substituting OH of a sulfo group with a monovalent group, R¹⁰ and R¹¹ may be bonded to each other to form a ring, X³ and X⁴ may be bonded to each other to form a ring, and Y⁻ is a monovalent anion.)
[3] The tetrazene radical salt compound according to [2], in which
   Y⁻ in the chemical formula (3) or the chemical formula (4) is a conjugate base of a strong acid or a conjugate base of a weak acid.
[4] A tetrazene-type alcohol oxidation catalyst which is an alcohol oxidation catalyst for oxidizing an alcohol, the catalyst containing:
   at least one selected from the group consisting of the tetrazene compound according to [1] and a derivative thereof.
[5] A tetrazene radical salt-type alcohol oxidation catalyst which is an alcohol oxidation catalyst for oxidizing an alcohol, the catalyst containing:
   at least one selected from the group consisting of the tetrazene radical salt compound according to [2] or [3] and a derivative thereof.
[6] A tetrazene-type alcohol oxidation catalyst which is an alcohol oxidation catalyst for oxidizing an alcohol, the catalyst containing:
   a tetrazene compound having a bicyclic skeleton represented by the following chemical formula (5): 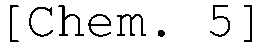 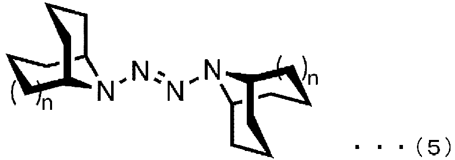
   (in the formula, n is 0 or 1).
[7] An alcohol oxidation method including:
   oxidizing an alcohol in presence of the tetrazene-type alcohol oxidation catalyst according to [4] and a cooxidant.
[8] The alcohol oxidation method according to [7], in which
   the alcohol is a primary alcohol or a secondary alcohol.
[9] The alcohol oxidation method according to [7] or [8], in which
   an addition amount of the tetrazene-type alcohol oxidation catalyst is 0.01 mol or more and 100 mol or less with respect to 100 mol of all hydroxyl groups in the alcohol.
[10] An alcohol oxidation method including:
   oxidizing an alcohol in presence of the tetrazene radical salt-type alcohol oxidation catalyst according to [5] and a cooxidant.
[11] The alcohol oxidation method according to [10], in which
   the alcohol is a primary alcohol or a secondary alcohol.
[12] The alcohol oxidation method according to [10] or [11], in which
   an addition amount of the tetrazene radical salt-type alcohol oxidation catalyst is 0.01 mol or more and 100 mol or less with respect to 100 mol of all hydroxyl groups in the alcohol.
[13] An alcohol oxidation method including:
   oxidizing an alcohol in presence of the tetrazene-type alcohol oxidation catalyst according to [6] and a cooxidant.
[14] The alcohol oxidation method according to [13], in which
   the alcohol is a primary alcohol or a secondary alcohol.
[15] The alcohol oxidation method according to [13] or [14], in which
   an addition amount of the tetrazene-type alcohol oxidation catalyst is 0.01 mol or more and 100 mol or less with respect to 100 mol of all hydroxyl groups in the alcohol.

### Advantageous Effects of Invention

According to the invention, new tetrazene compounds, new tetrazene radical salt compounds, new tetrazene-type alcohol oxidation catalysts, and new tetrazene radical salt-type alcohol oxidation catalysts that are each suitably applicable as alcohol oxidation catalysts capable of exhibiting a sufficiently high catalytic activity even in oxidation of secondary alcohols and that are easy to produce, and alcohol oxidation methods using them can be provided.

### Brief Description of Drawings

[FIG. 1] FIG. 1 is a diagram illustrating a measurement result of cyclic voltammetry of 1,2-di(2-azaadamantan-2-yl)diazene (DAD).
[FIG. 2] FIG. 2 is a diagram illustrating a measurement result of cyclic voltammetry of 2-azaadamantane N-oxyl (AZADO).

### Description of Embodiments

Hereinafter, the invention will be described in detail based on suitable embodiments thereof.

### [Tetrazene Compound having Adamantane Skeleton or Bicyclic Skeleton]

A tetrazene compound according to an embodiment of the invention will be described. The tetrazene compound of the present embodiment is a tetrazene compound having an adamantane skeleton represented by the following chemical formula (1) or a bicyclic skeleton represented by the following chemical formula (2).

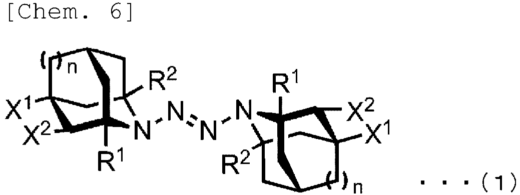

(In the formula (1), n is 0 or 1, R¹ is H, an alkyl group having 1 to 6 carbon atoms that may include a substituent, a phenyl group that may include a substituent, a carboxy group, an amide group, or an alkoxycarbonyl group having 1 to 6 carbon atoms, R² is H, an alkyl group having 1 to 6 carbon atoms that may include a substituent, a carboxy group, an amide group, or an alkoxycarbonyl group having 1 to 6 carbon atoms, X¹ is H, OR³ (R³ is H, an alkyl group having 1 to 6 carbon atoms that may include a substituent, an acyl group, or a silyl group having 3 to 18 carbon atoms), NR⁴R⁵ (R⁴ and R⁵ are each independently H, an alkyl group having 1 to 6 carbon atoms that may include a substituent, an acyl group, an alkoxycarbonyl group, or a group obtained by substituting OH of a sulfo group with a monovalent group, and R⁴ and R⁵ may be bonded to each other to form a ring), a halogen atom, a phenyl group that may include a substituent, or an alkyl group having 1 to 6 carbon atoms that may include a substituent, X² is H, OR³ (R³ is H, an alkyl group having 1 to 6 carbon atoms that may include a substituent, an acyl group, or a silyl group having 3 to 18 carbon atoms), NR⁴R⁵ (R⁴ and R⁵ are each independently H, an alkyl group having 1 to 6 carbon atoms that may include a substituent, an acyl group, an alkoxycarbonyl group, or a group obtained by substituting OH of a sulfo group with a monovalent group, and R⁴ and R⁵ may be bonded to each other to form a ring), or a halogen atom, and either X¹ or X² is H.)

In the formula (1), when n is 0, the skeleton is strictly a "noradamantane" skeleton, but such a skeleton is also included in the "adamantane skeleton" of the invention.

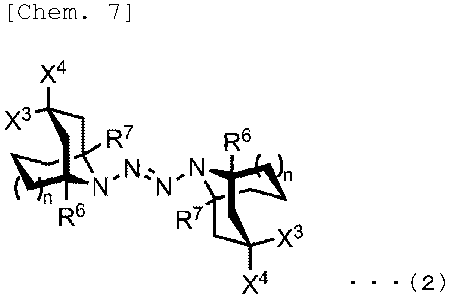

(In the formula (2), n is 0 or 1, R⁶ and R⁷ are each independently H or an alkyl group having 1 to 6 carbon atoms that may include a substituent, X³ and X⁴ are each independently H, an alkyl group having 1 to 6 carbon atoms that may include a substituent, an aromatic group that may include a substituent, OR³ (R³ is H, an alkyl group having 1 to 6 carbon atoms that may include a substituent, an acyl group, or a silyl group having 3 to 18 carbon atoms), NR⁴R⁵ (R⁴ and R⁵ are each independently H, an alkyl group having 1 to 6 carbon atoms that may include a substituent, an acyl group, an alkoxycarbonyl group, or a group obtained by substituting OH of a sulfo group with a monovalent group, and R⁴ and R⁵ may be bonded to each other to form a ring), or a halogen atom, or X³ and X⁴ may form a keto group, an imino group (NR⁸), an oxime group (NOR⁹), or a hydrazone group (NNR¹⁰R¹¹), the R⁸ to R¹¹ are each independently H, an alkyl group having 1 to 6 carbon atoms that may include a substituent, a phenyl group that may include a substituent, an acyl group, an alkoxycarbonyl group, or a group obtained by substituting OH of a sulfo group with a monovalent group, and R¹⁰ and R¹¹ may be bonded to each other to form a ring, X³ and X⁴ may be bonded to each other to form a ring, and one of R⁶, R⁷, X³, and X⁴ is not H.)

In the tetrazene compound having an adamantane skeleton represented by the above chemical formula (1), R¹ is preferably H, a methyl group, an ethyl group, an isopropyl group, an n-butyl group, a tert-butyl group, a phenyl group, an amide group, or a methoxycarbonyl group.

In the tetrazene compound having an adamantane skeleton represented by the above chemical formula (1), R² is preferably H, a methyl group, an ethyl group, an isopropyl group, an n-butyl group, a tert-butyl group, an amide group, or a methoxycarbonyl group.

In the tetrazene compound having an adamantane skeleton represented by the above chemical formula (1), X¹ is preferably H, a fluorine group, a chlorine group, a hydroxyl group, a methoxy group, an acetamide group, a 4-benzyloxyphenyl group, or a 3-acetoxypropyl group.

In the tetrazene compound having an adamantane skeleton represented by the above chemical formula (1), X² is preferably H, a fluorine group, a chlorine group, a methoxy group, or an acetamide group.

As for a combination of R¹, R² and X¹, X², a case where R¹, R², X¹, and X² are H, a case where R¹ is a methyl group and R², X¹, and X² are H, a case where R¹ and R² are a methyl group and X¹ and X² are H, a case where R¹, R², and X² are H and X¹ is a fluorine group, a case where R¹, R², and X² are H and X¹ is a hydroxyl group, a case where R¹, R², and X² are H and X¹ is a methoxy group, a case where R¹, R², and X¹ are H and X² is a fluorine group, and a case where R¹ is a methyl group, R² and X² are H, and X¹ is a fluorine group are preferred.

In the present description, the expression "silyl group having 3 to 18 carbon atoms" refers to a group represented by R^{a}₃Si- (R^{a} is any hydrocarbon group) in which a total number of carbon atoms in the hydrocarbon group is 3 to 18. Examples of the silyl group include trimethylsilyl, triethylsilyl, tert-butyldimethylsilyl, tert-butyldiphenylsilyl, triisopropylsilyl and triphenylsilyl, and among them, tert-butyldiphenylsilyl is preferred.

In the tetrazene compound having a bicyclic skeleton represented by the above chemical formula (2), R⁶ and R⁷ are each independently preferably H, a methyl group, an ethyl group, an isopropyl group, an n-butyl group, or a tert-butyl group.

In the tetrazene compound having a bicyclic skeleton represented by the above chemical formula (2), X³ and X⁴ are each independently preferably H, a methyl group, a 4-hydroxybutyl group, or an acetamide group. It is preferable that X³ and X⁴ form a keto group, an imino group (NR⁸), an oxime group (NOR⁹), or a hydrazone group (NNR¹⁰R¹¹). R⁸ is preferably a phenyl group, a tert-butoxycarbonyl group, or a benzenesulfonyl group. R⁹ is preferably a methyl group or a benzyl group. As for R¹⁰ and R¹¹, a case where R¹⁰ is H and R¹¹ is an acetyl group is preferred.

As for a combination of R⁶, R⁷ and X³, X⁴, a case where R⁶ and R⁷ are a methyl group and X³ and X⁴ are H, a case where R⁶, R⁷ and X⁴ are H and X³ is a methoxy group, a case where R⁶, R⁷ and X³ are H and X⁴ is an acetamide group, a case where R⁶ and R⁷ are H, X³ is a hydroxyl group, and X⁴ is a methyl group, and a case where R⁶ and R⁷ are H and X³ and X⁴ are a keto group are preferred.

The tetrazene compound of the present embodiment exhibits a higher catalytic activity in oxidation of a secondary alcohols when used as an alcohol oxidation catalyst and is also easy to produce. The tetrazene compound of the present embodiment exhibits a higher catalytic activity even in oxidation of a primary alcohol when used as an alcohol oxidation catalyst.

### [Method for Synthesizing Tetrazene Compounds having Adamantane Skeleton]

The tetrazene compounds represented by the above chemical formula (1) can be synthesized, for example, according to a reaction route shown in the following formula (6). Specifically, tetrazene can be synthesized by nitrosating and reducing a secondary amine to obtain N,N-dialkylhydrazine, and then dimerizing the N,N-dialkylhydrazine via aerobic oxidation in the presence of a copper catalyst.

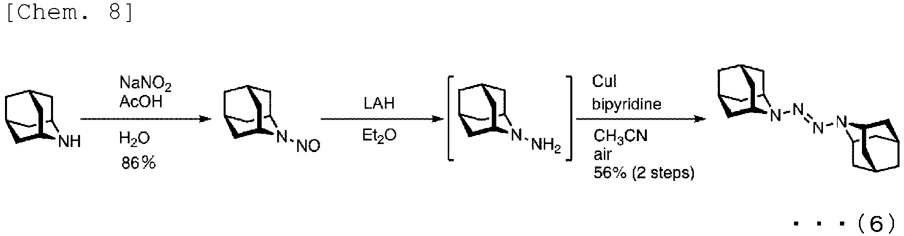

### [Tetrazene Radical Salt Compound having Adamantane Skeleton or Bicyclic Skeleton]

A tetrazene radical salt compound according to an embodiment of the invention will be described. The tetrazene radical salt compound of the present embodiment is a tetrazene radical salt compound having an adamantane skeleton represented by the following chemical formula (3) or a bicyclic skeleton represented by the following chemical formula (4).

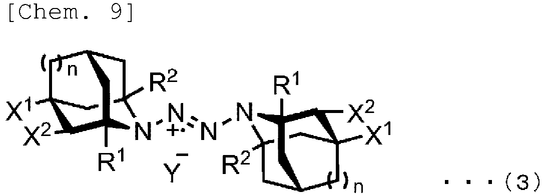

(In the formula (3), n is 0 or 1, R¹ is H, an alkyl group having 1 to 6 carbon atoms that may include a substituent, a phenyl group that may include a substituent, a carboxy group, an amide group, or an alkoxycarbonyl group having 1 to 6 carbon atoms, R² is H, an alkyl group having 1 to 6 carbon atoms that may include a substituent, a carboxy group, an amide group, or an alkoxycarbonyl group having 1 to 6 carbon atoms, X¹ is H, OR³ (R³ is H, an alkyl group having 1 to 6 carbon atoms that may include a substituent, an acyl group, or a silyl group having 3 to 18 carbon atoms), NR⁴R⁵ (R⁴ and R⁵ are each independently H, an alkyl group having 1 to 6 carbon atoms that may include a substituent, an acyl group, an alkoxycarbonyl group, or a group obtained by substituting OH of a sulfo group with a monovalent group, and R⁴ and R⁵ may be bonded to each other to form a ring), a halogen atom, a phenyl group that may include a substituent, or an alkyl group having 1 to 6 carbon atoms that may include a substituent, X² is H, OR³ (R³ is H, an alkyl group having 1 to 6 carbon atoms that may include a substituent, an acyl group, or a silyl group having 3 to 18 carbon atoms), NR⁴R⁵ (R⁴ and R⁵ are each independently H, an alkyl group having 1 to 6 carbon atoms that may include a substituent, an acyl group, an alkoxycarbonyl group, or a group obtained by substituting OH of a sulfo group with a monovalent group, and R⁴ and R⁵ may be bonded to each other to form a ring), or a halogen atom, either X¹ or X² is H, and Y⁻ is a monovalent anion.)

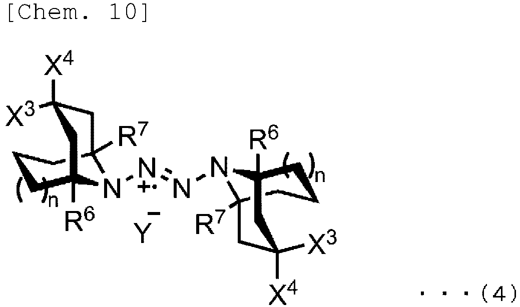

(In the formula (4), n is 0 or 1, R⁶ and R⁷ are each independently H or an alkyl group having 1 to 6 carbon atoms that may include a substituent, X³ and X⁴ are each independently H, an alkyl group having 1 to 6 carbon atoms that may include a substituent, an aromatic group that may include a substituent, OR³ (R³ is H, an alkyl group having 1 to 6 carbon atoms that may include a substituent, an acyl group, or a silyl group having 3 to 18 carbon atoms), NR⁴R⁵ (R⁴ and R⁵ are each independently H, an alkyl group having 1 to 6 carbon atoms that may include a substituent, an acyl group, an alkoxycarbonyl group, or a group obtained by substituting OH of a sulfo group with a monovalent group, and R⁴ and R⁵ may be bonded to each other to form a ring), or a halogen atom, or X³ and X⁴ may form a keto group, an imino group (NR⁸), an oxime group (NOR⁹), or a hydrazone group (NNR¹⁰R¹¹), the R⁸ to R¹¹ are each independently H, an alkyl group having 1 to 6 carbon atoms that may include a substituent, a phenyl group that may include a substituent, an acyl group, an alkoxycarbonyl group, or a group obtained by substituting OH of a sulfo group with a monovalent group, and R¹⁰ and R¹¹ may be bonded to each other to form a ring, X³ and X⁴ may be bonded to each other to form a ring, and Y⁻is a monovalent anion.)

In the tetrazene radical salt compound having an adamantane skeleton represented by the above chemical formula (3), R¹ is preferably H, a methyl group, an ethyl group, an isopropyl group, an n-butyl group, a tert-butyl group, a phenyl group, an amide group, or a methoxycarbonyl group.

In the tetrazene radical salt compound having an adamantane skeleton represented by the above chemical formula (3), R² is preferably H, a methyl group, an ethyl group, an isopropyl group, an n-butyl group, a tert-butyl group, an amide group, or a methoxycarbonyl group.

In the tetrazene radical salt compound having an adamantane skeleton represented by the above chemical formula (3), X¹ is preferably H, a fluorine group, a chlorine group, a hydroxyl group, a methoxy group, an acetamide group, a 4-benzyloxyphenyl group, or a 3-acetoxypropyl group.

In the tetrazene radical salt compound having an adamantane skeleton represented by the above chemical formula (3), X² is preferably H, a fluorine group, a chlorine group, a methoxy group, or an acetamide group.

As for a combination of R¹, R² and X¹, X², a case where R¹, R², X¹, and X² are H, a case where R¹ is a methyl group and R², X¹, and X² are H, a case where R¹ and R² are a methyl group and X¹ and X² are H, a case where R¹, R², and X² are H and X¹ is a fluorine group, a case where R¹, R², and X² are H and X¹ is a hydroxyl group, a case where R¹, R², and X² are H and X¹ is a methoxy group, a case where R¹, R², and X¹ are H and X² is a fluorine group, and a case where R¹ is a methyl group, R² and X² are H, and X¹ is a fluorine group are preferred.

In the tetrazene radical salt compound having a bicyclic skeleton represented by the above chemical formula (4), R⁶ and R⁷ are each independently preferably H, a methyl group, an ethyl group, an isopropyl group, an n-butyl group, or a tert-butyl group.

In the tetrazene radical salt compound having a bicyclic skeleton represented by the above chemical formula (4), X³ and X⁴ are each independently preferably H, a methyl group, a 4-hydroxybutyl group, or an acetamide group. It is preferable that X³ and X⁴ form a keto group, an imino group (NR⁸), an oxime group (NOR⁹), or a hydrazone group (NNR¹⁰R¹¹). R⁸ is preferably a phenyl group, a tert-butoxycarbonyl group, or a benzenesulfonyl group. R⁹ is preferably a methyl group or a benzyl group. As for R¹⁰ and R¹¹, a case where R¹⁰ is H and R¹¹ is an acetyl group is preferred.

As for a combination of R⁶, R⁷ and X³, X⁴, a case where R⁶ and R⁷ are a methyl group and X³ and X⁴ are H, a case where R⁶, R⁷ and X⁴ are H and X³ is a methoxy group, a case where R⁶, R⁷ and X³ are H and X⁴ is an acetamide group, a case where R⁶ and R⁷ are H, X³ is a hydroxyl group, and X⁴ is a methyl group, and a case where R⁶ and R⁷ are H and X³ and X⁴ are a keto group are preferred.

Examples of the silyl group include trimethylsilyl, triethylsilyl, tert-butyldimethylsilyl, tert-butyldiphenylsilyl, triisopropylsilyl and triphenylsilyl, and among them, tert-butyldiphenylsilyl is preferred.

Y⁻ in the chemical formula (3) or the chemical formula (4) is a monovalent anion, and examples thereof include a conjugate base of a strong acid or a conjugate base of a weak acid. Examples of the conjugate base of a strong acid include a tetrakis(3,5-bis(trifluoromethyl)phenyl)borate ion ([B(C₆H₃(CF₃)₂)₄]⁻) represented by the following chemical formula (7), a chloride ion (Cl⁻), a fluoride ion (F⁻), a tetrafluoroborate ion (BF₄⁻), a perchlorate ion (ClO₄⁻), a hexafluorophosphate ion (PF₆⁻), a hexafluoroantimonate ion (SbF₆⁻), and an alkyl sulfonate ion (RSO₃⁻, R is an alkyl group having 1 to 4 carbon atoms) such as a triflate ion (CF₃SO₃⁻). Examples of the conjugate base of a weak acid include a carboxylate ion (RCOO⁻, R is an alkyl group having 1 to 17 carbon atoms) such as acetate ion (CH₃COO⁻). Among them, the tetrakis(3,5-bis(trifluoromethyl)phenyl)borate ion is preferred from the viewpoint of solubility in an organic solvent and ease of purification by silica gel column chromatography.

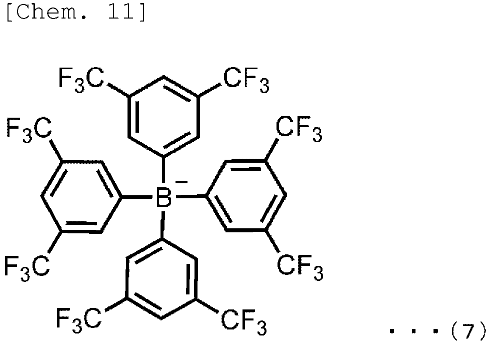

The tetrazene radical salt compound of the present embodiment exhibits a higher catalytic activity in oxidation of secondary alcohols when used as an alcohol oxidation catalyst and is also easy to produce. The tetrazene radical salt compound of the present embodiment is expected to exhibit a higher catalytic activity even in oxidation of a primary alcohol when used as an alcohol oxidation catalyst. The tetrazene radical salt compound of the present embodiment can be converted into the tetrazene compound of the above embodiment by reduction with a reducing agent such as calcium ascorbate. It is presumed that by utilizing this property, a reducing ability of a new reducing agent can be evaluated, and the reducing agent can be quantitatively evaluated.

### [Method for Synthesizing Tetrazene Radical Salt Compounds having Adamantane Skeleton]

The tetrazene radical salt compounds represented by the chemical formula (3) can be synthesized, for example, according to a reaction route shown in the following formula (8). Specifically, after tetrazene is subjected to one-electron oxidation by chlorine to form a tetrazene radical chloride, anion exchange with a tetrakis(3,5-bis(trifluoromethyl)phenyl)borate (BArF), enables isolation of the BArF salt of a tetrazene radical cation.

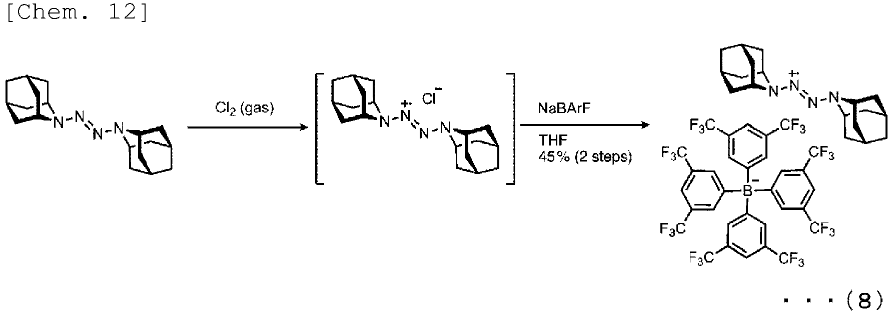

### [Tetrazene-type Alcohol Oxidation Catalyst]

### (First Embodiment)

A tetrazene-type alcohol oxidation catalyst according to an embodiment of the invention is an alcohol oxidation catalyst for oxidizing an alcohol, and contains at least one selected from the group consisting of the tetrazene compound of the above embodiment and a derivative thereof.

The tetrazene-type alcohol oxidation catalyst of the present embodiment contains at least one selected from the group consisting of the above tetrazene compound and a derivative thereof. The tetrazene-type alcohol oxidation catalyst of the present embodiment may contain one of the above tetrazene compound and the derivative thereof alone, or may contain two or more thereof in combination. Examples of the derivative of the tetrazene compound include a hydrate and a salt (hydrochloride and the like) of the tetrazene compound having an adamantane skeleton represented by the above formula (1) or the tetrazene compound having a bicyclic skeleton represented by the above formula (2).

The tetrazene-type alcohol oxidation catalyst of the present embodiment may simply contain the above tetrazene compound in an amount sufficient to serve as an effective catalyst, and may composed of at least one selected from the group consisting of the above tetrazene compound and the derivative thereof, or may further contain an impurity derived from a reagent used for the synthesis of the tetrazene compound, an impurity generated due to purification, and the like within a range not impairing the effects of the present invention.

### (Second Embodiment)

A tetrazene-type alcohol oxidation catalyst according to an embodiment of the invention is an alcohol oxidation catalyst for oxidizing an alcohol, and contains a tetrazene compound having a bicyclic skeleton represented by the following chemical formula (5).

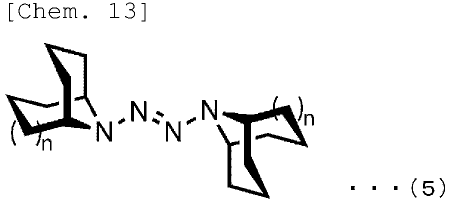

(In the formula, n is 0 or 1.)

### [Tetrazene Radical Salt-type Alcohol Oxidation Catalyst]

### (First Embodiment)

A tetrazene radical salt-type alcohol oxidation catalyst according to an embodiment of the invention is an alcohol oxidation catalyst for oxidizing an alcohol, and contains at least one selected from the group consisting of the tetrazene radical salt compound of the above embodiment and a derivative thereof.

The tetrazene radical salt-type alcohol oxidation catalyst of the present embodiment contains at least one selected from the group consisting of the above tetrazene radical salt compound and the derivative thereof. The tetrazene radical salt-type alcohol oxidation catalyst of the present embodiment may contain one of the above tetrazene radical salt compound and the derivative thereof alone, or may contain two or more thereof in combination.

The tetrazene radical salt-type alcohol oxidation catalyst of the present embodiment may simply contain the above tetrazene radical salt compound in an amount sufficient to serve as an effective catalyst, and may composed of at least one selected from the group consisting of the above tetrazene radical salt compound and the derivative thereof, or may further contain an impurity derived from a reagent used for the synthesis of the tetrazene radical salt compound, an impurity generated due to purification, and the like within a range not impairing the effects of the present invention.

### [Alcohol Oxidation Method]

### (First Embodiment)

An alcohol oxidation method according to an embodiment of the invention is a method of oxidizing an alcohol in presence of the tetrazene-type alcohol oxidation catalyst containing at least one selected from the group consisting of the tetrazene compound of the above embodiment and the derivative thereof and a cooxidant.

In the alcohol oxidation method of the present embodiment, the alcohol as a substrate may be a primary alcohol represented by the following chemical formula (9) or a secondary alcohol represented by the following chemical formula (10).

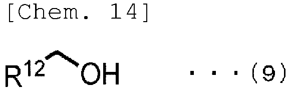

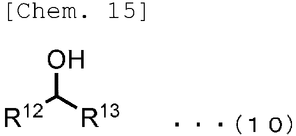

In the chemical formulae (9) and (10), R¹² and R¹³ each independently represent a substituent that does not adversely affect the oxidation reaction, and examples thereof include a linear or branched alkyl group that may be substituted, a cyclic alkyl group that may be substituted, an aromatic group that may be substituted, and a heterocyclic group that may be substituted. The alcohol according to the alcohol oxidation method of the present embodiment may be a polyhydric alcohol having a plurality of structural units represented by the above chemical formulae (9) and (10) in the same molecule, and is preferably a monohydric alcohol having one hydroxyl group from the viewpoint that the alcohol oxidation reaction proceeds more sufficiently.

Examples of the alkyl group in the "linear or branched alkyl group that may be substituted" include an alkyl group having 1 to 16 carbon atoms, and among them, an alkyl group having 1 to 8 carbon atoms is preferred. Examples of such an alkyl group include a methyl group, an ethyl group, an n-propyl group, an isopropyl group, an n-butyl group, an isobutyl group, an s-butyl group, a tert-butyl group, an n-pentyl group, an isopentyl group, a 2-methylbutyl group, a neopentyl group, a 1-ethylpropyl group, an n-hexyl group, an isohexyl group, a 4-methylpentyl group, a 3-methylpentyl group, a 2-methylpentyl group, a 1-methylpentyl group, a 3,3-dimethylbutyl group, a 2,2-dimethylbutyl group, a 1,1-dimethylbutyl group, a 1,2-dimethylbutyl group, a 1,3-dimethylbutyl group, a 2,3-dimethylbutyl group, a 2-ethylbutyl group, a heptyl group, a 1-methylhexyl group, a 2-methylhexyl group, a 3-methylhexyl group, a 4-methylhexyl group, a 2-methylheptyl group, a 3-methylheptyl group, a 4-methylheptyl group, a 5-methylheptyl group, a 6-methylheptyl group, a 1-propylpentyl group, a 2-ethylhexyl group, and a 5,5-dimethylhexyl group.

The substituent of the alkyl group is not particularly limited as long as it does not affect the oxidation reaction, and examples thereof include an alkyl group having 1 to 6 carbon atoms such as a methyl group, an ethyl group, and a propyl group; an alkoxy group having 1 to 6 carbon atoms such as a methoxy group, an ethoxy group, and a propoxy group; a halogen atom such as fluorine, chlorine, bromine, and iodine; an alkenyl group having 2 to 6 carbon atoms such as a vinyl group and an allyl group; an alkynyl group having 2 to 6 carbon atoms such as an ethynyl group and a propargyl group; a hydroxyl group; an amino group that may be substituted; a sulfonyl group that may be substituted; a cyano group; a nitroso group; an amidino group that may be substituted; a carboxy group; an alkoxycarbonyl group having 2 to 7 carbon atoms; a carbamoyl group that may be substituted; an aromatic group; an aromatic heterocyclic group; and an acyl group (an alkylcarbonyl group that may be substituted and an arylcarbonyl group that may be substituted).

Examples of the cyclic alkyl group in the "cyclic alkyl group that may be substituted" include a cycloalkyl group having 3 to 7 carbon atoms, such as a cyclopropyl group, a cyclobutyl group, a cyclopentyl group, a cyclohexyl group, and a cycloheptyl group. Examples of the substituent for the cyclic alkyl group include those same as the above substituents for the "alkyl group that may be substituted".

Examples of the aromatic group in the "aromatic group that may be substituted" include a monocyclic or fused polycyclic aromatic carbocyclic group, and specific examples thereof include an aryl group having 3 to 14 carbon atoms, such as a phenyl group, a naphthyl group, an anthryl group, an azulenyl group, a phenanthryl group, and an acenaphthylenyl group.

The heterocyclic ring in the "heterocyclic group that may be substituted" may be a 5-membered monocyclic ring, a 6-membered monocyclic ring, or an aromatic heterocyclic ring having a 6-5 or 6-6 fused ring structure and having 1 to 3 heteroatoms selected from the group consisting of an oxygen atom, a sulfur atom, and a nitrogen atom as heteroatoms. Specific examples of such a heterocyclic group include a monocyclic aromatic heterocyclic group such as a furyl group, a thienyl group, a pyrrolyl group, an oxazolyl group, an isoxazolyl group, a thiazolyl group, an isothiazolyl group, an imidazolyl group, a pyrazolyl group, a 1,2,3-oxadiazolyl group, a 1,2,4-oxadiazolyl group, a 1,3,4-oxadiazolyl group, a furazanyl group, a 1,2,3-thiadiazolyl group, a 1,2,4-thiadiazolyl group, a 1,3,4-thiadiazolyl group, a 1,2,3-triazolyl group, a 1,2,4-triazolyl group, a tetrazonyl group, a pyridyl group, a pyridazinyl group, a pyrimidinyl group, a pyrazinyl group, and a triazinyl group; and an 8-membered to 12-membered fused polycyclic aromatic heterocyclic group such as a benzofuranyl group, an isobenzoxazolyl group, a 1,2-benzisoxazolyl group, a benzothiazolyl group, a benzopyranyl group, a 1,2-benzisothiazolyl group, a 1H-benzotriazolyl group, a quinolyl group, an isoquinolyl group, a cinnolinyl group, a quinazolyl group, a quinoxalinyl group, a phthalazinyl group, a naphthyridinyl group, a purinyl group, a butyridinyl group, a carbazolinyl group, an α-carbolinyl group, a β-carbolinyl group, a γ-carbolinyl group, an acridinyl group, a phenoxazinyl group, a phenothiazinyl group, a phenazinyl group, a phenoxathiinyl group, a thianthrenyl group, a phenanthridinyl group, a phenanthrolinyl group, an indolizinyl group, a pyrrolo[1,2-b]pyridazinyl group, a pyrazolo[1,5-a]pyridyl group, an imidazo[1,2-a]pyridyl group, an imidazo[1,5-a]pyridyl group, an imidazo[1,2-b]pyridazinyl group, an imidazo[1,2-a]pyrimidinyl group, a 1,2,4-triazolo[4,3-a]pyridyl group, and a 1,2,4-triazolo[4,3-b]pyridazinyl group. Examples of the substituent for the aromatic group and the heterocyclic group include those same as the above substituents for the "alkyl group that may be substituted".

The alcohol oxidation method of the present embodiment may be a method in which the tetrazene-type alcohol oxidation catalyst of the above embodiment is added to the alcohol as a reaction substrate, or a method in which the alcohol is added to the tetrazene-type alcohol oxidation catalyst of the above embodiment.

An addition amount of the tetrazene-type alcohol oxidation catalyst is preferably 0.01 mol or more and 100 mol or less, and more preferably 0.1 mol or more and 10 mol or less, with respect to 100 mol of all hydroxyl groups in the alcohol. When the addition amount of the tetrazene-type alcohol oxidation catalyst is less than the lower limit, the reaction tends not to proceed, a reaction rate tends to decrease, or the reaction tends to stop in the middle. On the other hand, when the addition amount of the tetrazene-type alcohol oxidation catalyst exceeds the upper limit, it tends to be difficult to control the reaction rate. In the present embodiment, even when the addition amount of the tetrazene-type alcohol oxidation catalyst is small (for example, 0.1 mol or more and 1 mol or less with respect to 100 mol of all hydroxyl groups in the alcohol), a sufficiently high alcohol oxidation catalytic activity is exhibited.

In the alcohol oxidation method of the present embodiment, the alcohol is oxidized in presence of the tetrazene-type alcohol oxidation catalyst and the cooxidant. In the alcohol oxidation method of the present embodiment, the cooxidant oxidizes (catalytically oxidizes) the tetrazene-type alcohol oxidation catalyst, and then the oxidized tetrazene-type alcohol oxidation catalyst oxidizes the alcohol as a substrate, whereby the (substrate oxidation) reaction proceeds. In this reaction, the tetrazene-type alcohol oxidation catalyst is reduced with the oxidation reaction of the substrate. An oxidation cycle proceeds in which the reduced tetrazene-type alcohol oxidation catalyst is oxidized again by the cooxidant and the oxidized tetrazene-type alcohol oxidation catalyst oxidizes the substrate.

Such a cooxidant can be appropriately selected from, for example, those generally used in an oxidation reaction using TEMPO, and is not particularly limited, and examples thereof include peroxygenic acid, hydrogen peroxide, hypohalous acid and a salt thereof, perhalous acid and a salt thereof, a persulfate, a halide, a halogenating agent such as N-bromosuccinimide, trihalogenated isocyanuric acids, diacetoxyiodoarenes, oxygen, and a mixture thereof. Among them, as the cooxidant, peracetic acid, m-chloroperbenzoic acid, hydrogen peroxide, sodium hypochlorite, lithium hypochlorite, potassium hypochlorite, calcium hypochlorite, sodium hypobromite, lithium hypobromite, potassium hypobromite, calcium hypobromite, sodium hydrogen persulfate, sodium periodate, periodic acid, trichloroisocyanuric acid, tribromoisocyanuric acid, N-bromosuccinimide, N-chlorosuccinimide, chlorine, bromine, and iodine are preferred.

An addition amount of the cooxidant cannot be generalized as it depends on the type of the cooxidant, and generally, the addition amount of the cooxidant is preferably 33 mol or more, and more preferably 40 mol or more and 150 mol or less, with respect to 100 mol of all hydroxyl groups in the alcohol. For example, when sodium hypochlorite is used as the cooxidant, an addition amount of the sodium hypochlorite is preferably 100 mol or more with respect to 100 mol of all hydroxyl groups in the alcohol. When the addition amount of the cooxidant is less than the lower limit, the reaction tends not to proceed, the reaction rate tends to decrease, or the reaction tends to stop in the middle.

Further, for example, when the sodium hypochlorite is used as the cooxidant, it is preferable to further use other cooxidants such as an alkali metal halide and an additive such as a quaternary ammonium salt in combination for the purpose of further promoting the reaction. As used in combination with the sodium hypochlorite, tetrabutylammonium chloride, tetrabutylammonium bromide, sodium bromide, potassium bromide, and a mixture thereof are preferred. An addition amount thereof is preferably 10 mol or less with respect to 100 mol of the sodium hypochlorite.

Further, in the alcohol oxidation method of the present embodiment, a known solvent may be appropriately used within a range not impairing the effects of the present invention. Examples of such a solvent include aliphatic hydrocarbons such as hexane, heptane, and petroleum ether; aromatic hydrocarbons such as benzene, toluene, and xylene; nitriles such as acetonitrile and propionitrile; halogenated hydrocarbons such as dichloromethane, chloroform, 1,2-dichloroethane, and carbon tetrachloride; ethers such as diethyl ether, diisopropyl ether, tetrahydrofuran, dioxane, dimethoxyethane, and diethylene glycol dimethyl ether; amides such as formamide, dimethylformamide, dimethylacetamide, and hexamethylphosphoric acid triamide; sulfoxides such as dimethyl sulfoxide; esters such as ethyl formate, ethyl acetate, propyl acetate, butyl acetate, and diethyl carbonate; sulfolane; and water, and one of these may be used alone, or two or more thereof may be used in combination.

From the viewpoint that the reaction efficiency tends to be further improved, the solvent is preferably aliphatic hydrocarbons, aromatic hydrocarbons, nitriles, halogenated hydrocarbons, esters, water, or a mixture thereof, more preferably dichloromethane, acetonitrile, toluene, ethyl acetate, isopropyl acetate, water, or a mixed solution thereof, and still more preferably dichloromethane, acetonitrile, a dichloromethane-water mixed solution, an acetonitrile-water mixed solution, a toluene-water mixed solution, or an ethyl acetate-water mixed solution.

When a solvent is used, a concentration of the solvent is preferably 1 mL or more and 5 mL or less per 1 mmol of the alcohol.

In the alcohol oxidation method of the present embodiment, a buffer may be further added within a range not impairing the effects of the present invention. Examples of such a buffer include an alkali metal or alkaline earth metal carbonate, an alkali metal or alkaline earth metal bicarbonate, an alkali metal or alkaline earth metal hydroxide, an alkali metal or alkaline earth metal phosphate, and an alkali metal or alkaline earth metal acetate. One of these may be used alone, or two or more thereof may be used in combination. The buffer is preferably sodium hydrogen carbonate, sodium carbonate, sodium acetate, sodium dihydrogen phosphate, or disodium hydrogen phosphate. When the buffer is added, an addition amount thereof is preferably 5,000 mol or less with respect to 1 mol of the tetrazene compound contained in the alcohol oxidation catalyst.

A reaction temperature in the alcohol oxidation method of the present embodiment cannot be generalized as it depends on the types of the alcohol and the cooxidant, and is generally -80°C to 120°C and preferably 0°C to 40°C.

A reaction time can also not be generalized as it depends on the reaction temperature, and in the present embodiment, the reaction proceeds particularly quickly, and generally, the reaction time is preferably 0.3 hours to 12 hours.

By such an alcohol oxidation method of the present embodiment, the alcohol can be oxidized, and an aldehyde or a ketone corresponding to the alcohol can be obtained at a high yield. A reaction product obtained by oxidation can be isolated by an isolation operation such as extraction, recrystallization, and column chromatography after the completion of the reaction. Two or more of the isolation operations may be combined.

### [Alcohol Oxidation Method]

### (Second Embodiment)

An alcohol oxidation method according to an embodiment of the invention is a method of oxidizing an alcohol in presence of the tetrazene radical salt-type alcohol oxidation catalyst containing at least one selected from the group consisting of the tetrazene radical salt compound of the above embodiment and a derivative thereof and a cooxidant.

In the alcohol oxidation method of the present embodiment, as in the first embodiment, the alcohol as a substrate may be a primary alcohol represented by the above chemical formula (9) or a secondary alcohol represented by the above chemical formula (10).

The alcohol oxidation method of the present embodiment may be a method in which the tetrazene radical salt-type alcohol oxidation catalyst of the above embodiment is added to the alcohol as a reaction substrate, or a method in which the alcohol is added to the tetrazene radical salt-type alcohol oxidation catalyst of the above embodiment.

An addition amount of the tetrazene radical salt-type alcohol oxidation catalyst is preferably 0.01 mol or more and 100 mol or less, and more preferably 0.1 mol or more and 10 mol or less, with respect to 100 mol of all hydroxyl groups in the alcohol. When the addition amount of the tetrazene radical salt-type alcohol oxidation catalyst is less than the lower limit, the reaction tends not to proceed, a reaction rate tends to decrease, or the reaction tends to stop in the middle. On the other hand, when the addition amount of the tetrazene radical salt-type alcohol oxidation catalyst exceeds the upper limit, it tends to be difficult to control the reaction rate. In the present embodiment, even when the addition amount of the tetrazene radical salt-type alcohol oxidation catalyst is small (for example, 0.1 mol or more and 1 mol or less with respect to 100 mol of all hydroxyl groups in the alcohol), a sufficiently high alcohol oxidation catalytic activity is exhibited.

In the alcohol oxidation method of the present embodiment, as in the first embodiment, the alcohol is oxidized in presence of the tetrazene radical salt-type alcohol oxidation catalyst and the cooxidant.

Further, in the alcohol oxidation method of the present embodiment, a known solvent may be appropriately used as in the first embodiment within a range not impairing the effects of the present invention.

In the alcohol oxidation method of the present embodiment, a buffer may be further added as in the first embodiment within a range not impairing the effects of the present invention.

A reaction temperature in the alcohol oxidation method of the present embodiment cannot be generalized as it depends on the types of the alcohol and the cooxidant, and is generally -80°C to 120°C and preferably 0°C to 40°C.

A reaction time can also not be generalized as it depends on the reaction temperature, and in the present embodiment, the reaction proceeds particularly quickly, and generally, the reaction time is preferably 0.3 hours to 12 hours.

By such an alcohol oxidation method of the present embodiment, the alcohol can be oxidized, and an aldehyde or a ketone corresponding to the alcohol can be obtained at a high yield. A reaction product obtained by oxidation can be isolated by an isolation operation such as extraction, recrystallization, and column chromatography after the completion of the reaction. Two or more of the isolation operations may be combined.

### [Alcohol Oxidation Method]

### (Third Embodiment)

An alcohol oxidation method according to an embodiment of the invention is a method of oxidizing an alcohol in presence of the tetrazene-type alcohol oxidation catalyst containing the tetrazene compound having a bicyclic skeleton represented by the above chemical formula (5) of the above embodiment and a cooxidant.

In the alcohol oxidation method of the present embodiment, as in the first embodiment, the alcohol as a substrate may be a primary alcohol represented by the above chemical formula (9) or a secondary alcohol represented by the above chemical formula (10).

The alcohol oxidation method of the present embodiment may be a method in which the tetrazene-type alcohol oxidation catalyst of the above embodiment is added to the alcohol as a reaction substrate, or a method in which the alcohol is added to the tetrazene-type alcohol oxidation catalyst of the above embodiment.

An addition amount of the tetrazene-type alcohol oxidation catalyst is preferably 0.01 mol or more and 100 mol or less, and more preferably 0.1 mol or more and 10 mol or less, with respect to 100 mol of all hydroxyl groups in the alcohol. When the addition amount of the tetrazene-type alcohol oxidation catalyst is less than the lower limit, the reaction tends not to proceed, a reaction rate tends to decrease, or the reaction tends to stop in the middle. On the other hand, when the addition amount of the tetrazene-type alcohol oxidation catalyst exceeds the upper limit, it tends to be difficult to control the reaction rate. In the present embodiment, even when the addition amount of the tetrazene-type alcohol oxidation catalyst is small (for example, 0.1 mol or more and 1 mol or less with respect to 100 mol of all hydroxyl groups in the alcohol), a sufficiently high alcohol oxidation catalytic activity is exhibited.

In the alcohol oxidation method of the present embodiment, as in the first embodiment, the alcohol is oxidized in presence of the tetrazene-type alcohol oxidation catalyst and the cooxidant.

Further, in the alcohol oxidation method of the present embodiment, a known solvent may be appropriately used as in the first embodiment as long as the effects of the invention are not impaired.

Further, in the alcohol oxidation method of the present embodiment, a buffer may be further added as in the first embodiment within a range not impairing the effects of the present invention.

A reaction temperature in the alcohol oxidation method of the present embodiment cannot be generalized as it depends on the types of the alcohol and the cooxidant, and is generally -80°C to 120°C and preferably 0°C to 40°C.

A reaction time can also not be generalized as it depends on the reaction temperature, and in the present embodiment, the reaction proceeds particularly quickly, and generally, the reaction time is preferably 0.3 hours to 12 hours.

By such an alcohol oxidation method of the present embodiment, the alcohol can be oxidized, and an aldehyde or a ketone corresponding to the alcohol can be obtained at a high yield. A reaction product obtained by oxidation can be isolated by an isolation operation such as extraction, recrystallization, and column chromatography after the completion of the reaction. Two or more of the isolation operations may be combined.

### Example

The invention will be specifically described below with reference to Examples, and the invention is not limited to these Examples.

A compound obtained in each Example was analyzed by nuclear magnetic resonance spectroscopy (NMR: ¹H-NMR, ¹³C-NMR), infrared absorption spectroscopy (IR), mass spectrometry (MS), high-resolution mass spectrometry (electron ionization) (HRMS (EI)), and elemental analysis (Anal.). In ¹H-NMR, multiplicity is indicated as s = singlet, d = doublet, t = triplet, ddd = doublet of doublet of doublets, tt = triplet of triplets, dtd = doublet of triplet of doublets, dddd = doublet of doublet of doublet of doublets, and m = multiplet. J indicates a coupling constant. In each NMR, CDCl₃ was used as a solvent.

### (Example 1)

### <Synthesis of 1,2-Di(2-azaadamantan-2-yl)diazene>

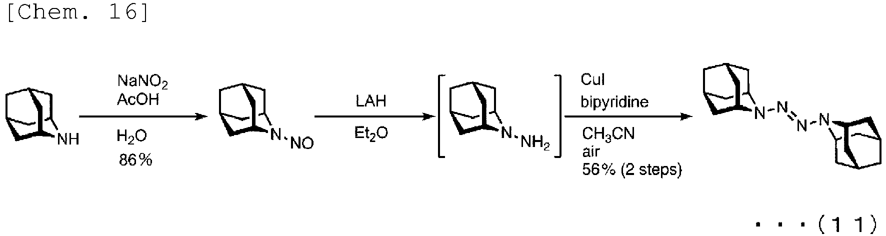

A 1,2-di(2-azaadamantan-2-yl)diazene (hereinafter referred to as DAD) compound represented by the following chemical formula (12) was synthesized according to a reaction route shown in the above formula (11). A starting material, 2-azaadamantane, was synthesized by a method reported by Shibuya et al. (Shibuya, M. et al., Synthesis, 2011, Vol. 21, pp. 3418-3425 (NPL 3)).

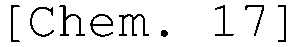

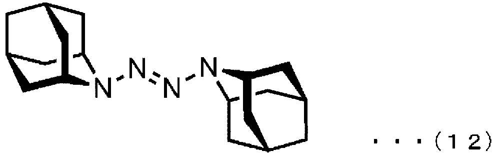

### <Synthesis of 2-Nitroso-2-azaadamantane>

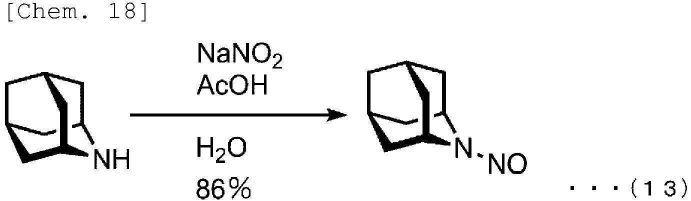

2-Nitroso-2-azaadamantane was synthesized according to a reaction route shown in the above formula (13). Specifically, first, acetic acid (0.33 mL, 5.5 mmol) and sodium nitrite (2.51 g, 36.4 mmol) were added at room temperature to a solution of 2-azaadamantane (500 mg, 3.64 mmol) in water (7.3 mL). This solution was stirred at 70 °C for 2 hours, and then cooled to room temperature (25 °C), and organic materials were extracted from the aqueous layer with diethyl ether. The organic layer was washed with saturated brine, then dried with sodium sulfate, and concentrated under reduced pressure. The obtained residue was purified by silica gel column chromatography to obtain a yellow solid of 2-nitroso-2-azaadamantane (520 mg, 3.13 mmol, yield 86%) in the fraction eluted with hexane-ethyl acetate (2:1 volume ratio).

The results of ¹H-NMR, ¹³C-NMR, IR, MS, and HRMS (EI) of the obtained 2-nitroso-2-azaadamantane are shown below.

¹H-NMR (400 MHz): δ 5.38 (s, 1H), 4.93 (s, 1H), 2.19 (s, 2H), 2.02 (s, 4H), 1.97-1.96 (m, 2H), 1.82-1.71 (m, 4H).
¹³C-NMR (100 MHz): δ: 55.2, 44.3, 36.8, 35.3, 34.9, 27.2.
IR (neat [cm⁻¹]): 2927, 2855.
MS [m/z]: 166 (M⁺), 136 (100%).
HRMS (EI): Calcd. for C₉H₁₄N₂O: 166.1106, found: 116.1108.

### <Synthesis of DAD>

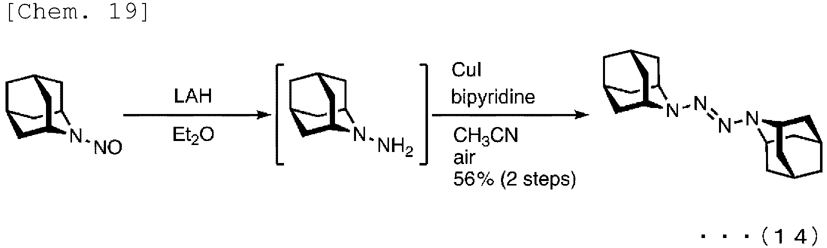

DAD was synthesized according to a reaction route shown in the above formula (14). Specifically, first, a solution of 2-nitroso-2-azaadamantane (200 mg, 1.20 mmol) in diethyl ether (Et₂O, 3.0 mL) was slowly added at 0 °C to a solution of lithium aluminum hydride (hereinafter referred to as LAH, 69 mg, 1.8 mmol) in diethyl ether (3.0 mL). This solution was stirred at room temperature for 2 hours, and then diethyl ether, water, and a 10% sodium hydroxide solution were each slowly added thereto at 0 °C, followed by stirring at room temperature (25 °C) for 1 hour. Thereafter, Celite filtration was performed, and concentration was performed under reduced pressure to obtain a white solid of 2-azaadamantan-2-amine (166 mg). The compound was used in a next reaction without further purification. Next, copper iodide (20.8 mg, 0.109 mmol) and 2,2'-bipyridine (20.4 mg, 0.131 mmol) were dissolved in acetonitrile (5.0 mL). To the solution, a solution of the previously synthesized 2-azaadamantan-2-amine (166 mg) in acetonitrile (6.0 mL) was slowly added dropwise at 0 °C over 3 hours. Thereafter, the mixture was stirred in open air at room temperature (25 °C) for 12 hours, and then tetramethylethylenediamine (32 µL, 0.22 mmol) was added thereto, followed by stirring for several minutes. Water was added to the solution, the aqueous layer was extracted with dichloromethane and discarded, and the organic layer was further washed with saturated brine, then dried with sodium sulfate, and concentrated under reduced pressure. The obtained residue was purified by silica gel column chromatography to obtain a white solid of DAD (101 mg, 0.336 mmol, yield 56% (2 steps)) in the fraction eluted with hexane-ethyl acetate (4:1 volume ratio).

The results of ¹H-NMR, ¹³C-NMR, IR, MS, and HRMS (EI) of the obtained DAD are shown below.

¹H-NMR (400 MHz): δ 4.07 (s, 4H), 2.02 (s, 4H), 2.00 (d, J = 10.6 Hz, 8H), 1.84 (s, 4H), 1.66 (d, J = 10.6 Hz, 8H).
¹³C-NMR (100 MHz): δ: 51.1, 36.5, 34.0, 27.4. IR (neat [cm⁻¹]): 2922, 2846.
MS [m/z]: 300 (M⁺), 80 (100%).
HRMS (EI): Calcd. for C₁₈H₂₈N₄: 300.2314, found: 300.2308.

### (Example 2)

### <Synthesis of DAD Radical Salt (BArF Salt)>

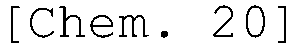

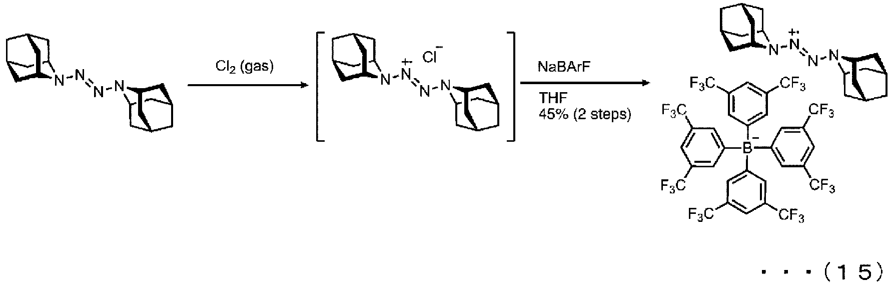

A DAD radical salt (BArF salt) represented by the following chemical formula (16) was synthesized according to a reaction route shown in the above formula (15). A starting material, DAD represented by the above chemical formula (12), was synthesized according to a reaction route shown in the above formula (11).

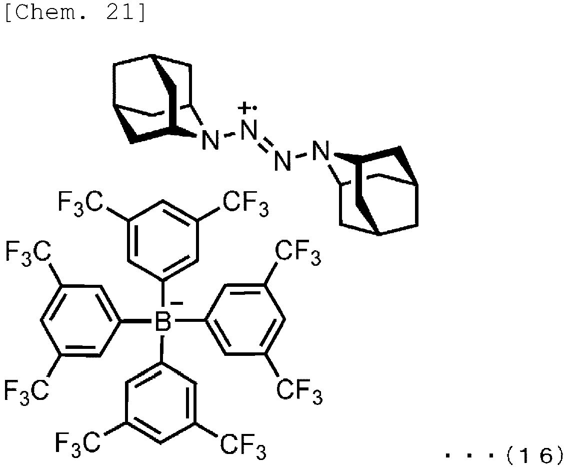

### <Synthesis of DAD Radical Salt (BArF Salt)>

A DAD radical salt (BArF salt) represented by the above chemical formula (16) was synthesized according to the reaction route shown in the above formula (15). Specifically, first, a solution of DAD (100 mg, 0.332 mmol) in carbon tetrachloride (3.3 mL) was bubbled with chlorine gas at room temperature (25 °C) (the chlorine gas was generated by adding concentrated hydrochloric acid dropwise to manganese dioxide under heating conditions). Thereafter, the precipitated red solid was collected by filtration, washed with diethyl ether, and then dried to obtain a red solid of DAD radical salt (chloride) (89 mg). The compound was used in a next reaction without further purification. Next, sodium tetrakis[3,5-bis(trifluoromethyl)phenyl]borate hydrate (247 mg, 0.279 mmol) was added at room temperature to a solution of DAD radical salt (chloride) (89 mg) in tetrahydrofuran (2.7 mL). This solution was stirred at room temperature (25 °C) for 2 hours, and then water was added thereto. Organic materials were extracted from the aqueous layer with dichloromethane. The organic layers were dried with sodium sulfate and concentrated under reduced pressure. The obtained residue was purified by silica gel column chromatography to obtain a red solid of DAD radical cation (BArF salt) (175 mg, 0.150 mmol, 45% (2 steps)) in the fraction eluted with chloroform-methanol (20:1 volume ratio).

The results of IR and elemental analysis of the obtained DAD radical salt (BArF salt) compound are shown below.

IR (neat [cm⁻¹]): 3078, 3021, 2943, 2866, 1794, 1608, 1449.
Anal. Calcd. for C₅₀H₄₀BF₂₄N₄: for C, 51.61; H, 3.46; N, 4.81; Found: C, 51.63; H, 3.61; N, 4.81.

### <Single Crystal X-ray Structure Analysis of DAD Radical Salt (BArF Salt)>

Using the DAD radical salt (BArF salt) synthesized in Example 2, a single crystal was prepared by a vapor diffusion method using chloroform as a good solvent and n-hexane as a poor solvent.

The obtained single crystal was selected and subjected to an X-ray diffraction experiment.

As a result, it was found that the present crystal belongs to a monoclinic system and has a space group Cc, a lattice constant a = 15.0246 (13) Å, b = 13.8861 (10) Å, c = 23.3019 (19) Å, β = 96.968(3)°, and Z = 4, and 33792 pieces of reflection data were measured.

Structural analysis was performed as follows. A phase was determined by a direct method (SHELXL), and a non-hydrogen atom position was found by Fourier synthesis.

A hydrogen atom position bonded to carbon was calculated from a carbon atom position.

### (Example 3)

### <Synthesis of 1,2-Bis(1-methyl-2-azaadamantan-2-yl)diazene>

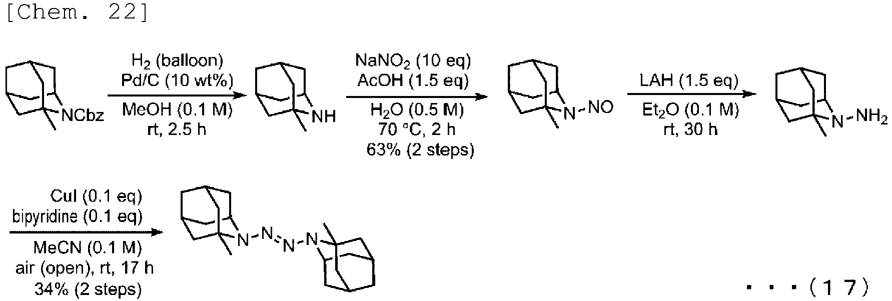

1,2-Bis(1-methyl-2-azaadamantan-2-yl)diazene (hereinafter referred to as "1-methyl-DAD") represented by the following chemical formula (18) was synthesized according to a reaction route shown in the above formula (17). A starting material, benzyl 1-methyl-2-azaadamantane-2-carboxylate, was synthesized by a method reported by Shibuya et al. (Shibuya, M. et al., Synthesis, 2011, No. 21, pp. 3418-3425 (NPL 3)).

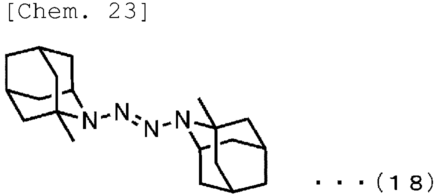

### <Synthesis of 1-Methyl-2-nitroso-2-azaadamantane>

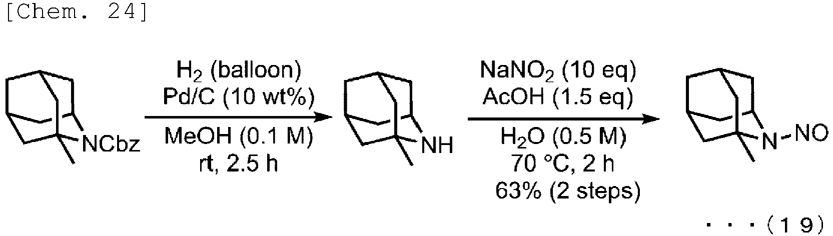

1-Methyl-2-nitroso-2-azaadamantane was synthesized according to a reaction route shown in the above formula (19). Specifically, first, palladium on activated carbon (Pd 10%) (12.2 mg) was added at room temperature to a solution of benzyl 1-methyl-2-azaadamantane-2-carboxylate (122 mg, 426 µmol) in methanol (4.3 mL) in a reaction vessel purged with argon gas. The vessel was filled with hydrogen gas (1 atm), followed by stirring at room temperature for 2.5 hours. Thereafter, the reaction vessel was purged with argon gas, and the reaction solution was subjected to Celite filtration. The obtained filtrate was concentrated under reduced pressure. Dichloromethane and a saturated aqueous sodium carbonate solution were added to the obtained residue, and organic materials were extracted from the aqueous layer with dichloromethane. The organic layers were dried with potassium carbonate and concentrated under reduced pressure to obtain a pale yellow oil of 1-methyl-2-azaadamantane (80 mg). The compound was used in a next reaction without further purification. Next, acetic acid (45.7 µL, 796 µmol) and sodium nitrite (366 mg, 5.31 mmol) were added at room temperature to a solution of the obtained pale yellow oil (80 mg) in water (1.1 mL). This solution was stirred at 70 °C for 2 hours, and then cooled to room temperature, and organic materials were extracted from the aqueous layer with diethyl ether. The organic layers were washed with saturated brine, then dried with sodium sulfate, and concentrated under reduced pressure. The obtained residue was purified by silica gel column chromatography to obtain a yellow solid of 1-methyl-2-nitroso-2-azaadamantane (48.7 mg, 63% (2 steps)) in the fraction eluted with hexane-ethyl acetate (5:1 volume ratio to 2:1 volume ratio).

The results of ¹H-NMR, ¹³C-NMR, IR, MS, and HRMS (EI) of the obtained 1-methyl-2-nitroso-2-azaadamantane are shown below.

¹H-NMR (400 MHz): δ 5.51 (s, 1H), 2.17 (s, 2H), 1.94-1.81 (m, 6H), 1.76 (d, J = 12.8 Hz, 2H), 1.68-1.58 (m, 5H).
¹³C-NMR (100 MHz): δ 60.0, 44.2, 43.9, 34.8, 34.5, 27.2, 26.5.
IR (neat [cm⁻¹]): 2929, 1410, 1333, 1242, 1122.
MS [m/z]: 180 (M⁺), 56 (100%).
HRMS (EI): Calcd. for C₁₃H₁₆N₂O: 180.1263, found: 180.1266.

### <Synthesis of 1-Methyl-DAD>

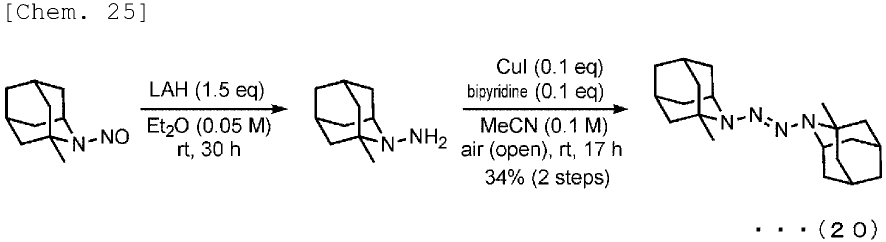

1-Methyl-DAD was synthesized according to a reaction route shown in the above formula (20). Specifically, first, a solution of 1-methyl-2-nitroso-2-azaadamantane (120 mg, 666 µmol) in diethyl ether (Et₂O, 3.3 mL) was slowly added at 0 °C to a solution of LAH (38 mg, 1.0 mmol) in diethyl ether (3.3 mL). This solution was stirred at room temperature for 29.5 hours, and then diethyl ether, water, and a 10% sodium hydroxide solution were each slowly added thereto at 0 °C, followed by stirring at room temperature for 15 minutes. Thereafter, Celite filtration was performed, and concentration was performed under reduced pressure to obtain a white solid (108 mg) containing 1-methyl-2-azaadamantan-2-amine. The compound was used in a next reaction without further purification. Next, copper iodide (12.4 mg, 65.0 µmol) and 2,2'-bipyridine (10.1 mg, 65.0 µmol) were dissolved in acetonitrile (1.9 mL). Separately, acetonitrile (2.2 mL) was added to the previously synthesized white solid (108 mg) containing 1-methyl-2-azaadamantan-2-amine. A solution portion thereof was slowly added dropwise to the acetonitrile solution of copper iodide and 2,2'-bipyridine over 3 hours at room temperature, and the undissolved solid was collected (since the solid was confirmed to contain 1-methyl-DAD, the solid was mixed with an organic layer after the subsequent extraction step was completed and purified by silica gel column chromatography). Thereafter, the mixture was stirred in open air at room temperature for 17 hours, and then tetramethylethylenediamine (19.6 µL, 130 µmol) was added thereto, followed by stirring for 10 minutes. Water was added to the solution, and organic materials were extracted from the aqueous layer with dichloromethane. The organic layers were further washed with saturated brine, then dried with sodium sulfate, and concentrated under reduced pressure. A mixture of the obtained residue and the undissolved solid was purified by silica gel column chromatography to obtain a white solid of 1-methyl-DAD (36.9 mg, 112 µmol, yield 34% (2 steps)) in the fraction eluted with hexane-ethyl acetate (8:1 volume ratio to 4:1 volume ratio).

The results of ¹H-NMR, ¹³C-NMR, IR, MS, and HRMS (EI) of the obtained 1-methyl-DAD are shown below.

¹H-NMR (400 MHz): δ 4.37 (s, 2H), 2.02 (s, 4H), 1.94 (d, J = 12.2 Hz, 4H), 1.84-1.67 (m, 8H), 1.56 (s, 8H), 1.24 (s, 6H).
¹³C-NMR (100 MHz): δ 55.8, 48.7, 42.5, 35.9, 32.5, 27.9, 27.8.
IR (neat [cm⁻¹]): 2958, 2912, 2846, 1442.
MS [m/z]: 328 (M⁺), 94 (100%).
HRMS (EI): Calcd. for C₂₀H₃₂N₄: 328.2627, found: 328.2639.

### (Example 4)

### <Synthesis of 1-Methyl-DAD Radical Salt (BArF Salt)>

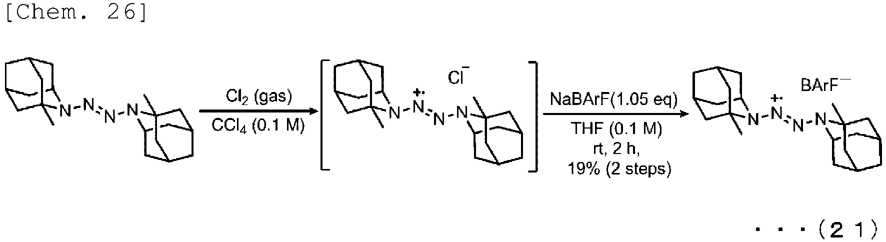

A 1-methyl-DAD radical salt (BArF salt) represented by the following chemical formula (22) was synthesized according to a reaction route shown in the above formula (21). A starting material, 1-methyl-DAD represented by the above chemical formula (18), was synthesized according to a reaction route shown in the above formula (17).

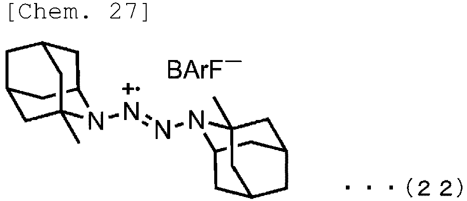

### <Synthesis of 1-Methyl-DAD Radical Salt (BArF Salt)>

A 1-methyl-DAD radical salt (BArF salt) represented by the above formula (22) was synthesized according to the reaction route shown in the above formula (21). Specifically, first, a solution of 1-methyl-DAD (158 mg, 0.48 mmol) in carbon tetrachloride (4.8 mL) was bubbled with chlorine gas at room temperature (the chlorine gas is generated by adding concentrated hydrochloric acid dropwise to manganese dioxide under heating conditions). Thereafter, the precipitated red solid was collected by filtration and washed with carbon tetrachloride. The obtained red solid was dried under reduced pressure to obtain a red solid (168 mg) of 1-methyl-DAD radical salt (chloride). The compound was used in a next reaction without further purification. Next, sodium tetrakis[3,5-bis(trifluoromethyl)phenyl]borate hydrate (430 mg, 0.49 mmol) was added at 0 °C to a solution of 1-methyl-DAD radical salt (chloride) (168 mg) in tetrahydrofuran (4.6 mL). This solution was stirred at room temperature for 2 hours, and then water was added thereto. After organic materials were extracted from the aqueous layer with dichloromethane, the organic layers were dried with sodium sulfate and concentrated under reduced pressure. The obtained residue was purified by silica gel column chromatography to obtain a red solid of 1-methyl-DAD radical salt (BArF salt) (107 mg, 89.9 µmol, 19% (2 steps)) in the fraction eluted with chloroform-methanol (20:1 volume ratio).

The results of elemental analysis of the obtained 1-methyl-DAD radical salt (BArF salt) are shown below.

Anal. Calcd. for C₅₂H₄₄BF₂₄N₄: for C, 52.41; H, 3.72; N, 4.70; found: C, 52.46; H, 3.86; N, 4.65.

### (Example 5)

### <Synthesis of 1,2-Bis(5-methoxy-2-azaadamantan-2-yl)diazene>

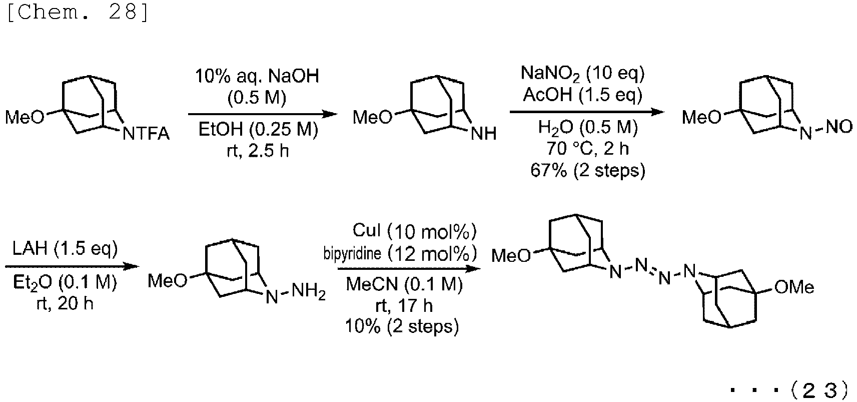

1,2-Bis(5-methoxy-2-azaadamantan-2-yl)diazene (hereinafter referred to as "5-methoxy-DAD") represented by the following chemical formula (24) was synthesized according to a reaction route shown in the above formula (23). A starting material, 2,2,2-trifluoro-1-(5-methoxy-2-azaadamantanyl)ethanone, was synthesized by a method reported by Shibuya et al. (Shibuya, M. et al., J. Org. Chem., 2014, Vol. 79, No. 21, pp. 10256-10268).

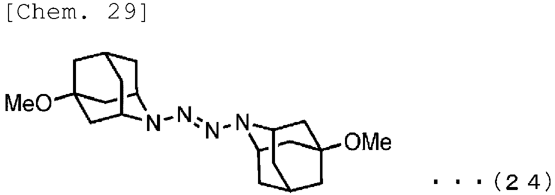

### <Synthesis of 5-Methoxy-2-nitroso-azaadamantane>

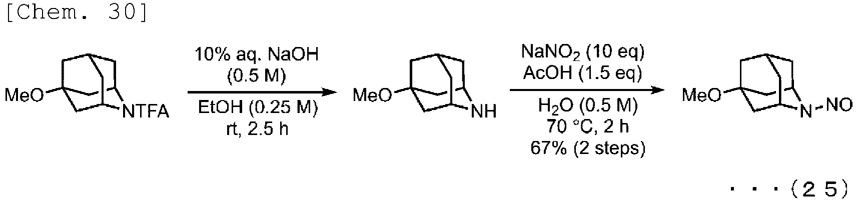

5-Methoxy-2-nitroso-2-azaadamantane was synthesized according to a reaction route shown in the above formula (25). First, a 10% aqueous sodium hydroxide solution (4.8 mL) was added to a solution of 2,2,2-trifluoro-1-(5-methoxy-2-azaadamantanyl)-1-ethanone (601 mg, 2.28 mmol) in ethanol (7.1 mL), followed by stirring at room temperature for 2.5 hours. Thereafter, the mixture was concentrated under reduced pressure, chloroform was added to the obtained residue, and organic materials were extracted from the aqueous layer with chloroform. The organic layers were dried with potassium carbonate and concentrated under reduced pressure to obtain a colorless oil of 5-methoxy-2-azaadamantane (370 mg). The compound was used in a next reaction without further purification. Next, acetic acid (191 µL, 3.32 mmol) and sodium nitrite (1.53 g, 22.1 mmol) were added at room temperature to a solution of the obtained colorless oil (370 mg) in water (4.4 mL). This solution was stirred at 70 °C for 1.5 hours, and then cooled to room temperature, and organic materials were extracted from the aqueous layer with diethyl ether. The organic layers were washed with saturated brine, then dried with sodium sulfate, and concentrated under reduced pressure. The obtained residue was purified by silica gel column chromatography to obtain a yellow solid of 5-methoxy-2-nitroso-2-azaadamantane (298 mg, 67% (2 steps)) in the fraction eluted with hexane-ethyl acetate (4:1 volume ratio to 2:1 volume ratio).

The results of ¹H-NMR, ¹³C-NMR, IR, MS, and HRMS (EI) of the obtained 5-methoxy-2-nitroso-2-azaadamantane are shown below.

¹H-NMR (400 MHz): δ 5.49 (s, 1H), 5.12 (s, 1H), 3.24 (s, 3H), 2.44 (s, 1H), 2.03-1.81 (m, 6H), 1.78-1.56 (m, 4H).
¹³C-NMR (100 MHz): δ 71.2, 56.1, 48.3, 45.0, 40.1, 39.2, 38.3, 35.8, 33.9, 29.2
IR (neat [cm⁻¹]): 3581, 3498, 2939, 2860, 1431.
MS [m/z]: 196 (M⁺), 94 (100%).
HRMS (EI): Calcd. for C₁₀H₁₆N₂O₂: 196.1212, found: 196.1207.

### <Synthesis of 5-Methoxy-DAD>

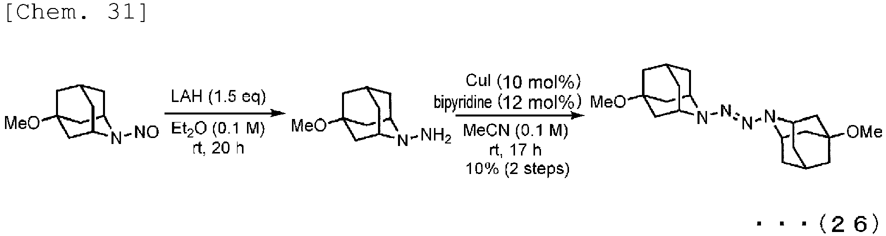

5-Methoxy-DAD was synthesized according to a reaction route shown in the above formula (26). Specifically, first, a solution of 5-methoxy-2-nitroso-2-azaadamantane (200 mg, 1.02 mmol) in diethyl ether (Et₂O, 5.1 mL) was slowly added at 0 °C to a solution of LAH (58.0 mg, 1.53 mmol) in diethyl ether (5.1 mL). This solution was stirred at room temperature for 24 hours, and then diethyl ether, water, and a 10% sodium hydroxide solution were each slowly added thereto at 0 °C, followed by stirring at room temperature for 15 minutes. Thereafter, Celite filtration was performed, and concentration was performed under reduced pressure to obtain a white solid (185 mg) containing 5-methoxy-2-azaadamantan-2-amine. The compound was used in a next reaction without further purification. Next, copper iodide (19.4 mg, 102 µmol) and 2,2'-bipyridine (19.1 mg, 122 µmol) were dissolved in acetonitrile (4.3 mL). To the solution, a solution of the previously synthesized white solid (185 mg) containing 5-methoxy-2-azaadamantan-2-amine in acetonitrile (5.1 mL) was slowly added dropwise at 0 °C over 3 hours. Thereafter, the mixture was stirred in open air at room temperature for 17 hours, and then tetramethylethylenediamine (30.0 µL, 204 µmol) was added thereto, followed by stirring for 10 minutes. Water was added to the solution, and organic materials were extracted from the aqueous layer with dichloromethane. The organic layers were further washed with saturated brine, then dried with sodium sulfate, and concentrated under reduced pressure. The obtained residue was purified by silica gel column chromatography to obtain a white solid of 5-methoxy-DAD (18.9 mg, 52.3 µmol, yield 10% (2 steps)) in the fraction eluted with hexane-ethyl acetate (3:1 volume ratio).

The results of ¹H-NMR, ¹³C-NMR, IR, MS, and HRMS (EI) of the obtained 5-methoxy-DAD are shown below.

¹H-NMR (400 MHz): δ 4.30 (s, 4H), 3.22 (s, 6H), 2.29 (s, 2H), 1.90 (t, J = 10.7 Hz, 8H), 1.82 (s, 4H), 1.69-1.50 (m, 8H).
¹³C-NMR (100 MHz): δ 71.7, 52.8, 47.8, 39.9, 37.2, 33.3, 29.3.
IR (neat [cm⁻¹]): 2937, 2852, 1109, 1088, 1065.
MS [m/z]: 360 (M⁺), 275 (100%).
HRMS (EI): Calcd. for C₂₀H₃₂N₄O₂: 360.2525, found: 360.2521.

### (Example 6)

### <Synthesis of 1,2-Bis(4-fluoro-2-azaadamantan-2-yl)diazene>

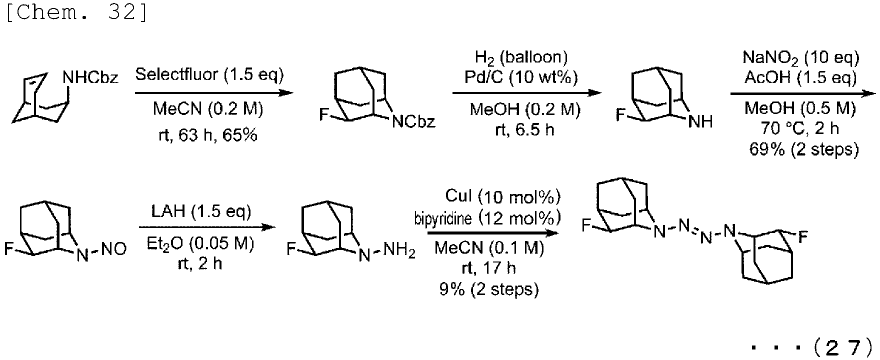

1,2-Bis(4-fluoro-2-azaadamantan-2-yl)diazene (hereinafter referred to as "4-fluoro-DAD") represented by the following chemical formula (28) was synthesized according to a reaction route shown in the above formula (27). A starting material, benzyl bicyclo[3.3.1]nonenyl carbamate, was synthesized using a method reported by Nagasawa et al. (Shota Nagasawa et al., Asian J. Org. Chem., 2023, Vol. 12, No. 4, e202300031).

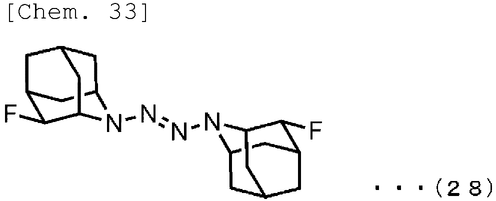

### <Synthesis of Benzyl 4-fluoro-2-azaadamantane-2-carboxylate>

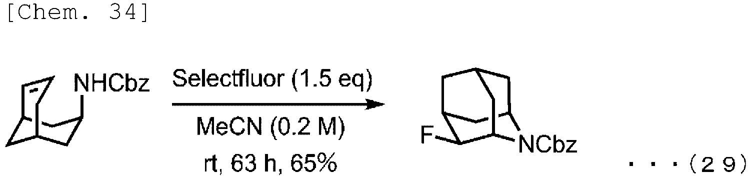

Benzyl 4-fluoro-2-azaadamantane-2-carboxylate was synthesized according to a reaction route shown in the above formula (29). Specifically, first, selectfluor (783 mg, 2.21 mmol) was added at 0 °C to a solution of benzyl bicyclo[3.3.1]nonenyl carbamate (400 mg, 1.47 mmol) in acetonitrile (7.4 mL). This solution was warmed to room temperature and stirred for 68.5 hours. Thereafter, the reaction solution was diluted with water, and organic materials were extracted from the aqueous layer with dichloromethane. The organic layers were further washed with saturated brine, then dried with magnesium sulfate, and concentrated under reduced pressure. The obtained residue was purified by silica gel column chromatography to obtain a pale yellow oily mixture (279 mg) containing benzyl 4-fluoro-2-azaadamantane-2-carboxylate as a main component in the fraction eluted with hexane-ethyl acetate (8:1 volume ratio).

The results of ¹H-NMR of the obtained benzyl 4-fluoro-2-azaadamantane-2-carboxylate are shown below.

¹H-NMR (400 MHz): δ 7.36-7.31 (m, 5H), 5.14 (s, 2H), 4.69-4.57 (m, 1H), 4.47-4.26 (m, 2H), 2.24-1.60 (m, 10H).

### <Synthesis of 4-Fluoro-2-nitroso-2-azaadamantane>

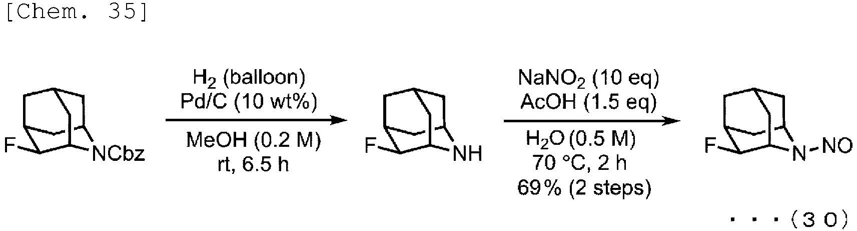

4-Fluoro-2-nitroso-2-azaadamantane was synthesized according to a reaction route shown in the above formula (30). Specifically, first, palladium on activated carbon (Pd 10%) (27.9 mg) was added at room temperature to a solution of the pale yellow oily mixture (279 mg) containing benzyl 4-fluoro-2-azaadamantane-2-carboxylate as a main component in methanol (4.8 mL) in a reaction vessel purged with argon gas. The vessel was filled with hydrogen gas (1 atm), followed by stirring at room temperature for 6.5 hours. Thereafter, the reaction vessel was purged with argon gas, and the reaction solution was subjected to Celite filtration. The obtained filtrate was washed with a 10% aqueous sodium hydroxide solution, and then the organic layer was dried with potassium carbonate and concentrated under reduced pressure to obtain a pale yellow oil (137 mg) containing 4-fluoro-2-azaadamantane as a main component. The compound was used in a next reaction without further purification. Next, acetic acid (76.0 µL, 1.32 mmol) and sodium nitrite (608 mg, 8.81 mmol) were added at room temperature to a solution of the obtained pale yellow oil (137 mg) in water (1.8 mL). This solution was stirred at 70 °C for 2 hours, and then cooled to room temperature, and organic materials were extracted from the aqueous layer with diethyl ether. The organic layers were washed with saturated brine, then dried with sodium sulfate, and concentrated under reduced pressure. The obtained residue was purified by silica gel column chromatography to obtain a yellow solid mixture (123 mg, 69% (2 steps)) containing 4-fluoro-2-nitroso-2-azaadamantane as a main component in the fraction eluted with hexane-ethyl acetate (4:1 volume ratio).

The results of ¹H-NMR of the obtained 4-fluoro-2-nitroso-2-azaadamantane are shown below.

¹H-NMR (400 MHz): δ 5.46 (s, 0.5H), 5.27 (s, 0.5H), 5.05 (s, 0.5H), 4.93 (s, 0.5H), 4.74 (dt, J = 50, 3.9 Hz, 0.5H), 4.41 (dt, J = 50, 3.9 Hz, 0.5H), 2.46-1.50 (m, 10H).

### <Synthesis of 4-Fluoro-DAD>

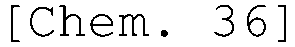

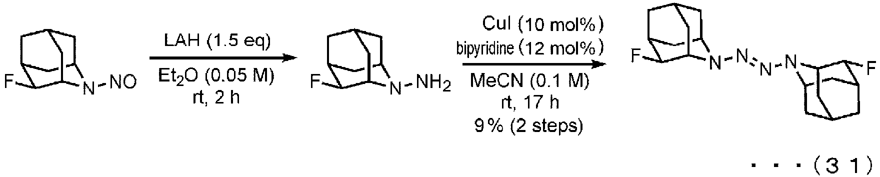

4-Fluoro-DAD was synthesized according to a reaction route shown in the above formula (31). Specifically, first, a solution of the yellow solid mixture (123 mg, 666 µmol) containing 4-fluoro-2-nitroso-2-azaadamantane as a main component in diethyl ether (Et₂O, 1.1 mL) was slowly added at 0 °C to a solution of LAH (38 mg, 1.0 mmol) in diethyl ether (1.1 mL). This solution was stirred at room temperature for 2 hours, and then diethyl ether, water, and a 10% sodium hydroxide solution were each slowly added thereto at 0 °C, followed by stirring at room temperature for 15 minutes. Thereafter, Celite filtration was performed, and concentration was performed under reduced pressure to obtain a white solid (112 mg) containing 4-fluoro-2-azaadamantan-2-amine. The compound was used in a next reaction without further purification. Next, copper iodide (12.5 mg, 65.8 µmol) and 2,2'-bipyridine (10.3 mg, 65.8 µmol) were dissolved in acetonitrile (3.0 mL). To the solution, a solution of the previously synthesized white solid (112 mg) containing 4-fluoro-2-azaadamantan-2-amine in acetonitrile (3.6 mL) was slowly added dropwise at room temperature over 2 hours and 40 minutes. Thereafter, the mixture was stirred in open air at room temperature for 12 hours, and then tetramethylethylenediamine (9.9 µL, 65.8 µmol) was added thereto, followed by stirring for 10 minutes. Water was added to the solution, and organic materials were extracted from the aqueous layer with dichloromethane. The organic layers were further washed with saturated brine, then dried with sodium sulfate, and concentrated under reduced pressure. The obtained residue was purified by silica gel column chromatography using hexane-ethyl acetate (15:1 volume ratio to 10:1 volume ratio) as an eluent, and then purified by preparative thin layer chromatography using toluene as an eluent, thereby obtaining a white solid of 4-fluoro-DAD (10.5 mg, 31.2 µmol, yield: 9% (2 steps)).

The results of ¹H-NMR, ¹³C-NMR, IR, MS, and HRMS (EI) of the obtained 4-fluoro-DAD are shown below.

¹H-NMR (400 MHz): δ 4.70 (d, J = 50 Hz, 2H), 4.12 (d, J = 78 Hz, 4H), 2.26 (s, 2H), 2.12 (d, J = 14 Hz, 2H) 2.06-1.87 (m, 8H), 1.86-1.77 (m, 4H), 1.73-1.59 (m, 4H).
¹³C-NMR (100 MHz): δ 90.8 (d, J = 4.0 Hz), 53.3, 53.0, 33.9, 33.8, 32.4 (d, J = 2.0 Hz), 32.3 (d, J = 2.0 Hz), 32.1, 32.0, 30.1, 28.2, 26.0.
IR (neat [cm⁻¹]): 2929, 2856, 1007, 957.
MS [m/z]: 336 (M⁺), 79 (100%).
HRMS (EI): Calcd. for C₁₈H₂₆F₂N₄: 336.2126, found: 336.2127.

### (Example 7)

### <Synthesis of 1,2-Di(9-azanoradamantan-9-yl)diazene>

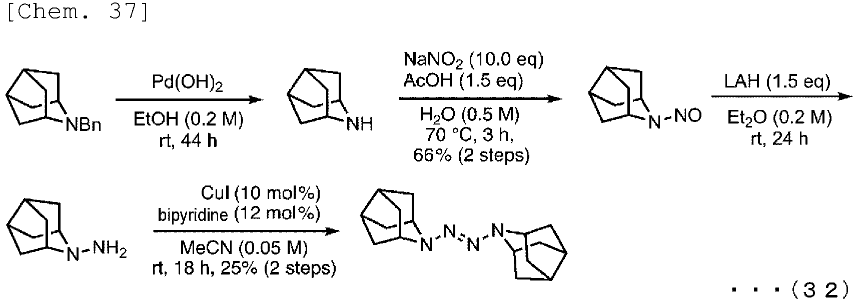

1,2-Di(9-azanoradamantan-9-yl)diazene (hereinafter referred to as "Nor-DAD") represented by the following chemical formula (33) was synthesized according to a reaction route shown in the above formula (32). A starting material, 9-benzyl-9-azanoradamantane, was synthesized by a method reported by Hayashi et al. (Hayashi, M. et al., Chem. Pharm. Bull., 2011, Vol. 59, No. 12, pp. 1570-1573 (NPL 4)).

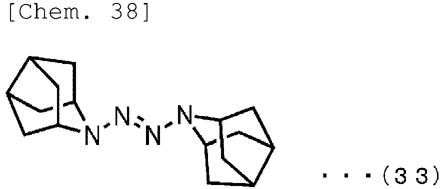

### <Synthesis of 9-Nitroso-9-azanoradamantane>

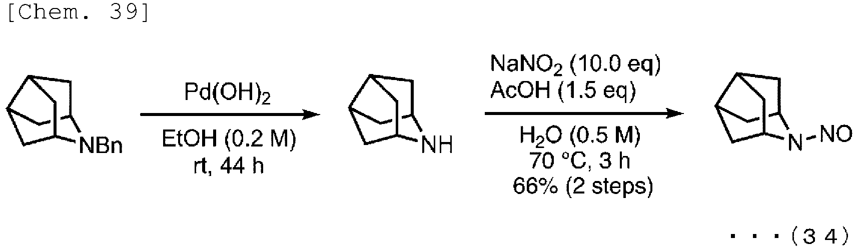

9-Nitroso-9-azanoradamantane was synthesized according to a reaction route shown in the above formula (34). Specifically, first, palladium hydroxide on activated carbon (Pd 20%, approximately 50% hydrated) (50 mg) was added at room temperature to a solution of 9-benzyl-9-azanoradamantane (500 mg, 2.34 mmol) in ethanol (12 mL) in a reaction vessel purged with argon gas. The reaction vessel was filled with hydrogen gas (1 atm), followed by stirring at room temperature for 44 hours. Thereafter, the reaction vessel was purged with argon gas, and the reaction solution was subjected to Celite filtration. The obtained filtrate was concentrated under reduced pressure to obtain a white solid of 9-azanoradamantane (355 mg). The compound was used in a next reaction without further purification. Next, acetic acid (260 µL, 4.32 mmol) and sodium nitrite (1.99 g, 28.8 mmol) were added at room temperature to a solution of the obtained white solid (355 mg) in water (6.0 mL). This solution was stirred at 70 °C for 3 hours, and then cooled to room temperature, and an organic layer was extracted from the aqueous layer with diethyl ether. The organic layer was washed with saturated brine, then dried with sodium sulfate, and concentrated under reduced pressure. The obtained residue was purified by silica gel column chromatography to obtain a yellow solid of 9-nitroso-9-azanoradamantane (250 mg, 66% (2 steps)) in the fraction eluted with hexane-ethyl acetate (20:1 volume ratio).

The results of ¹H-NMR, ¹³C-NMR, IR, MS, and HRMS (EI) of the obtained 7-nitroso-azanoradamantane are shown below.

¹H-NMR (400 MHz): δ 5.60 (s, 1H), 5.17 (s, 1H), 2.87 (t, J = 4.8 Hz, 2H), 1.94-1.85 (m, 2H), 1.81-1.70 (m, 5H), 1.52 (s,1H).
¹³C-NMR (100 MHz): δ 63.9, 52.7, 42.6, 41.8, 36.3.
IR (neat [cm⁻¹]): 2973, 2920, 2879, 1456, 1418, 1354, 1321, 1308, 1274, 1237, 1179, 1076, 1040, 987, 964, 917, 788, 764.
MS [m/z] : 152 (M⁺), 95 (100%).
HRMS (EI): Calcd. for C₈H₁₂N₂O: 152.0950, found: 152.0949.

### <Synthesis of Nor-DAD>

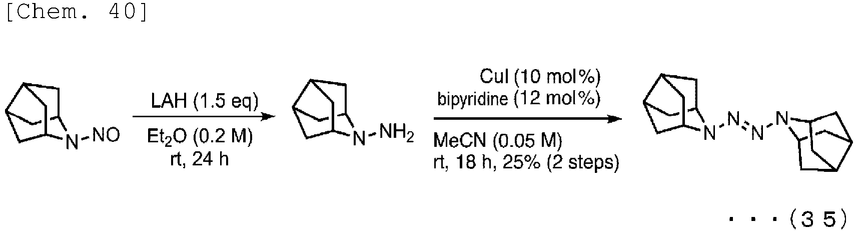

Nor-DAD was synthesized according to a reaction route shown in the above formula (35). Specifically, first, a solution of 9-nitroso-9-azanoradamantane (220 mg, 1.45 mmol) in diethyl ether (Et₂O, 4.0 mL) was slowly added at 0°C to a solution of LAH (82.3 mg, 2.17 mmol) in diethyl ether (3.0 mL). This solution was stirred at room temperature for 24 hours, and then diethyl ether, water, and a 10% sodium hydroxide solution were each slowly added thereto at 0 °C, followed by stirring at room temperature for 30 minutes. Thereafter, Celite filtration was performed, and concentration was performed under reduced pressure to obtain a mixture of 9-nitroso-9-azanoradamantane, 9-azanoradamantan-9-amine, and Nor-DAD. The mixture was purified by silica gel column chromatography to collect 9-nitroso-9-azanoradamantane (105 mg). The collected starting material was again subjected to reduction using LAH in accordance with the above procedure to obtain 9-azanoradamantan-9-amine (63 mg) and Nor-DAD (17 mg) in total of the two reactions. Since the obtained 9-azanoradamantan-9-amine was unstable in the air, the obtained 9-azanoradamantan-9-amine was immediately used in a next reaction. Next, copper iodide (8.7 mg, 46 µmol) and 2,2'-bipyridine (8.5 mg, 55 µmol) were dissolved in acetonitrile (2 mL). To the solution, a solution of the previously synthesized 9-azanoradamantan-9-amine (63 mg) in acetonitrile (6.0 mL) was slowly added dropwise at room temperature over 3.5 hours. Thereafter, the mixture was stirred in open air at room temperature for 18 hours, and then tetramethylethylenediamine (12 µL, 91 µmol) was added thereto, followed by stirring for 10 minutes. Water was added to the solution, and organic materials were extracted from the aqueous layer with dichloromethane. The organic layers were further washed with saturated brine, then dried with sodium sulfate, and concentrated under reduced pressure. The obtained residue was purified by silica gel column chromatography to obtain a white solid of Nor-DAD (33 mg) in the fraction eluted with hexane-ethyl acetate (10:1 volume ratio). A total yield amount and yield percentage, including the Nor-DAD (17 mg) obtained in the previous step, were 50 mg, 0.18 mmol, and yield 25% (2 steps).

The results of ¹H-NMR, ¹³C-NMR, IR, MS, and HRMS (EI) of the obtained Nor-DAD are shown below.

¹H-NMR (400 MHz): δ 4.34 (s, 4H), 2.60 (t, J = 4.8 Hz, 4H), 1.73 (d, J = 10.4 Hz, 8H), 1.64-1.55 (m, 8H).
¹³C-NMR (100 MHz): δ 60.6, 40.7, 36.5.
IR (neat [cm⁻¹]): 2965, 2909, 2867, 1450, 1353, 1319, 1211, 1058, 1040, 1031, 964, 943, 903, 783.
MS [m/z]: 272 (M⁺), 67 (100%).
HRMS (EI): Calcd. for C₁₆H₂₄N₄: 272.2001, found: 272.2003.

### (Example 8)

### <Synthesis of Nor-DAD Radical Salt (BArF Salt)>

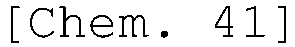

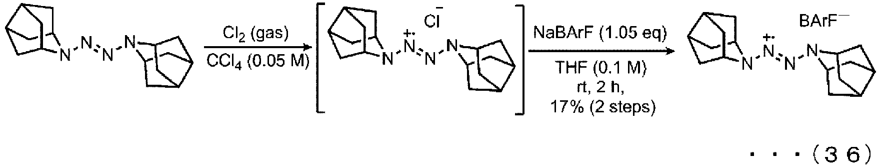

A Nor-DAD radical salt (BArF salt) represented by the following chemical formula (37) was synthesized according to a reaction route shown in the above formula (36). A starting material, Nor-DAD represented by the above chemical formula (35), was synthesized according to a reaction route shown in the above formula (32).

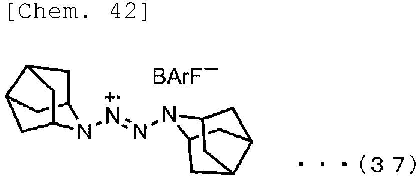

### <Synthesis of Nor-DAD Radical Salt (BArF Salt)>

A Nor-DAD radical salt (BArF salt) represented by the above chemical formula (37) was synthesized according to the reaction route shown in the above formula (36). Specifically, first, a solution of Nor-DAD (40 mg, 0.15 mmol) in carbon tetrachloride (3.0 mL) was bubbled with chlorine gas at room temperature (the chlorine gas is generated by adding concentrated hydrochloric acid dropwise to manganese dioxide under heating conditions). Thereafter, the precipitated red solid was collected by filtration and washed with carbon tetrachloride. The obtained red solid was dried under reduced pressure for 2 hours to obtain a red solid (31 mg) of Nor-DAD radical salt (chloride). The compound was used in a next reaction without further purification. Next, sodium tetrakis[3,5-bis(trifluoromethyl)phenyl]borate hydrate (94 mg, 0.11 mmol) was added at 0°C to a solution of Nor-DAD radical salt (chloride) (31 mg) in tetrahydrofuran (1.0 mL). This solution was stirred at room temperature for 2 hours, and then water was added thereto. After organic materials were extracted from the aqueous layer with dichloromethane, the organic layers were dried with sodium sulfate and concentrated under reduced pressure. The obtained residue was purified by silica gel column chromatography to obtain a red solid (20 mg, 17 µmol, 17% (2 steps)) of Nor-DAD radical salt (BArF salt) in the fraction eluted with chloroform-methanol (20:1 volume ratio).

The results of elemental analysis of the obtained Nor-DAD radical salt (BArF salt) are shown below.

Anal. Calcd. for C₄₈H₄₀BF₂₄N₄: for C, 50.77; H, 3.20; N, 4.93; found: C, 50.79; H, 3.37; N, 4.87.

### (Example 9)

### <Synthesis of 1,2-Di(9-azabicyclo[3.3.1]nonan-9-yl)diazene>

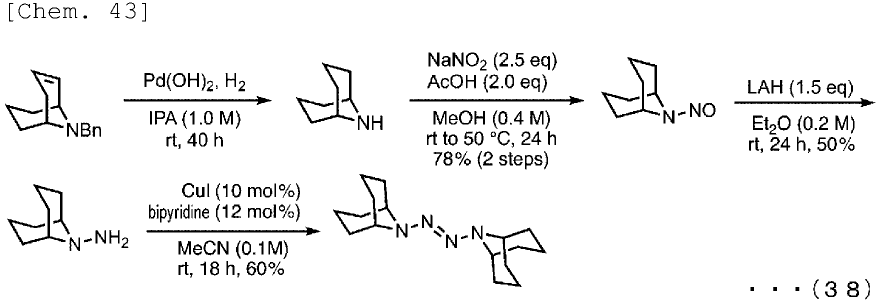

1,2-Di(9-azabicyclo[3.3.1]nonan-9-yl)diazene (hereinafter referred to as "DAND") represented by the following chemical formula (39) was synthesized according to a reaction route shown in the above formula (38). A starting material, 9-benzyl-9-azabicyclo[3.3.1]nonene, was synthesized using a method reported by Song et al. (Zhiguo J. Song et al., Organic Syntheses, 2020, Vol. 99, pp. 251-273) .

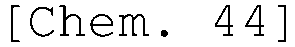

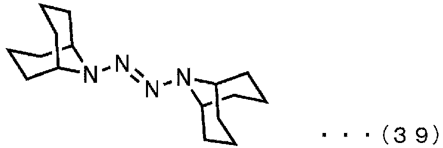

### <Synthesis of 9-Nitroso-9-azabicyclo[3.3.1]nonane>

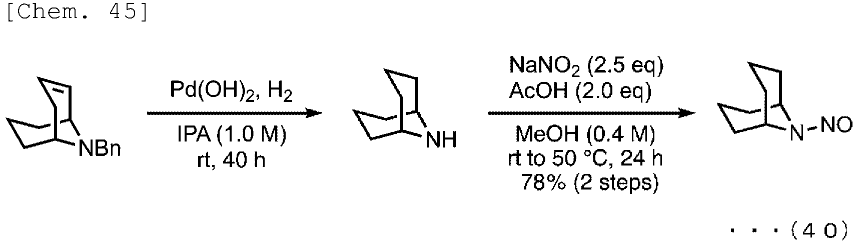

9-Nitroso-9-azabicyclo[3.3.1]nonane was synthesized according to a reaction route shown in the above formula (40). Specifically, first, palladium hydroxide on activated carbon (Pd 20%, approximately 50% hydrated) (150 mg) was added at room temperature to a solution of 9-benzyl-9-azabicyclo[3.3.1]nonene (1.50 g, 7.00 mmol) in isopropanol (7.0 mL) in a reaction vessel purged with argon gas. The reaction vessel was filled with hydrogen gas (1 atm), followed by stirring at room temperature for 40 hours. Thereafter, the reaction vessel was purged with argon gas, and the reaction solution was subjected to Celite filtration. The obtained filtrate was concentrated under reduced pressure to obtain a white solid (880 mg) of 9-azabicyclo[3.3.1]nonane. The compound was used in a next reaction without further purification. Next, acetic acid (0.84 mL, 14.1 mmol) and sodium nitrite (1.21 g, 17.6 mmol) were added at room temperature to a solution of the obtained white solid (880 mg) in methanol (18 mL). This solution was stirred at room temperature for 10 hours and then further stirred at 50 °C for 14 hours. Thereafter, the mixture was cooled to room temperature, and organic materials were extracted from an aqueous layer with diethyl ether. The organic layers were washed with saturated brine, then dried with sodium sulfate, and concentrated under reduced pressure. The obtained residue was purified by silica gel column chromatography to obtain a yellow solid of 9-nitroso-9-azabicyclo[3.3.1]nonane (842 mg, 78% (2 steps)) in the fraction eluted with hexane-ethyl acetate (5:1 volume ratio).

The results of ¹H-NMR, ¹³C-NMR, IR, MS, and HRMS (EI) of the obtained 9-nitroso-9-azabicyclo[3.3.1]nonane are shown below.

¹H-NMR (400 MHz): δ 5.31-5.24 (m, 1H), 4.85 (s, 1H), 2.29-1.87 (m, 7H), 1.80-1.55 (m, 5H).
¹³C-NMR (100 MHz): δ 54.3, 43.2, 31.2, 29.2, 20.0.
IR (neat [cm⁻¹]): 2947, 2916, 2854, 1423, 1369, 1277, 1192, 1111, 1018, 914, 733.
MS [m/z] : 154 (M⁺), 96 (100%).
HRMS (EI): Calcd. for C₃H₁₄N₂O: 154.1106, found: 154.1106.

### <Synthesis of 9-Azabicyclo[3.3.1]nonan-9-amine>

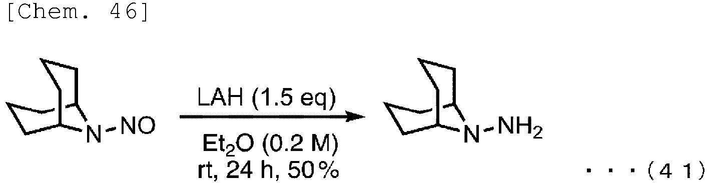

9-azabicyclo[3.3.1]nonan-9-amine was synthesized according to a reaction route shown in the above formula (41). Specifically, first, a solution of 9-nitroso-9-azabicyclo[3.3.1]nonane (400 mg, 2.59 mmol) in diethyl ether (Et₂O, 6.0 mL) was slowly added at 0 °C to a solution of LAH (148 mg, 3.89 mmol) in diethyl ether (7.0 mL). This solution was stirred at room temperature for 24 hours, and then diethyl ether, water, and a 10% sodium hydroxide solution were each slowly added thereto at 0 °C, followed by stirring at room temperature for 30 minutes. Thereafter, Celite filtration was performed, and concentration was performed under reduced pressure. The obtained residue was purified by silica gel column chromatography to obtain a white solid of 9-azabicyclo[3.3.1]nonan-9-amine (181 mg, 50%) in the fraction eluted with chloroform-methanol (1:1 volume ratio).

The results of ¹H-NMR, ¹³C-NMR, IR, MS, and HRMS (EI) of the obtained 9-azabicyclo[3.3.1]nonan-9-amine are shown below.

¹H-NMR (400 MHz): δ 2.95 (s, 2H), 2.17-2.06 (m, 5H), 1.98-1.85 (m, 3H), 1.60-1.52 (m, 6H).
¹³C-NMR (100 MHz): δ 55.8, 19.7.
IR (neat [cm⁻¹]): 3323, 2923, 1608, 1454, 1312, 1122, 1041, 897, 801.
MS [m/z]: 140 (M⁺), 97 (100%).
HRMS (EI): Calcd. for C₈H₁₆N₂: 140.1313, found: 140.1307.

### <Synthesis of DAND>

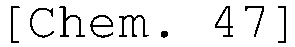

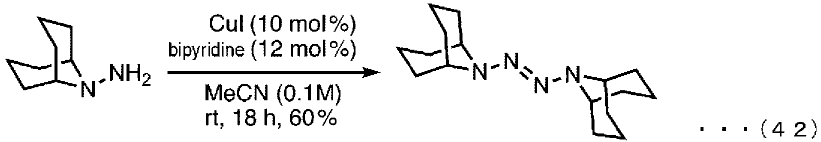

DAND was synthesized according to a reaction route shown in the above formula (42). Specifically, first, copper iodide (20.4 mg, 107 µmol) and 2,2'-bipyridine (20.0 mg, 128 µmol) were dissolved in acetonitrile (5 mL). To the solution, a solution of the previously synthesized 9-azabicyclo[3.3.1]nonan-9-amine (150 mg, 1.07 mmol) in acetonitrile (6 mL) was slowly added dropwise at room temperature over 2.5 hours. Thereafter, the mixture was stirred in open air at room temperature for 18 hours, and then tetramethylethylenediamine (32 µL, 0.21 mmol) was added thereto, followed by stirring for 10 minutes. Water was added to the solution, and organic materials were extracted from the aqueous layer with dichloromethane. The organic layers were further washed with saturated brine, then dried with sodium sulfate, and concentrated under reduced pressure. The obtained residue was purified by silica gel column chromatography to obtain a white solid of DAND (88 mg, 0.32 mmol, yield 60%) in the fraction eluted with hexane-ethyl acetate (20:1 volume ratio).

The results of ¹H-NMR, ¹³C-NMR, IR, MS, and HRMS (EI) of the obtained DAND are shown below.

¹H-NMR (400 MHz): δ 4.04 (s, 4H), 2.15-1.95 (m, 12H), 1.66-1.56 (m, 12H).
¹³C-NMR (100 MHz): δ 50.4, 28.2, 20.8.
IR (neat [cm⁻¹]): 2927, 2846, 1435, 1371, 1304, 1248, 1078, 1012, 928, 895, 804, 752.
MS [m/z]: 276 (M⁺), 96 (100%).
HRMS (EI): Calcd. for C₁₆H₂₈N₄: 276.2314, found: 276.2326.

### (Example 10)

### <Synthesis of DAND Radical Salt (BArF Salt)>

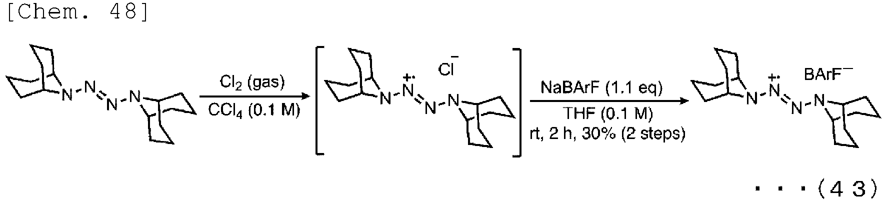

A DAND radical salt (BArF salt) represented by the following chemical formula (44) was synthesized according to a reaction route shown in the above formula (43). A starting material, DAND represented by the above chemical formula (39), was synthesized according to a reaction route shown in the above formula (38).

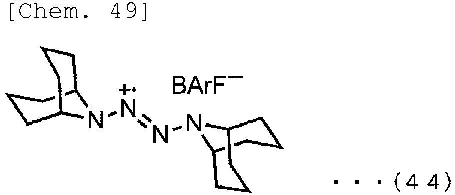

### <Synthesis of DAND Radical Salt (BArF Salt)>

A DAND radical salt (BArF salt) represented by the above chemical formula (44) was synthesized according to the reaction route shown in the above formula (43). Specifically, first, a solution of DAND (80 mg, 0.29 mmol) in carbon tetrachloride (3.0 mL) was bubbled with chlorine gas at room temperature (the chlorine gas was generated by adding concentrated hydrochloric acid dropwise to manganese dioxide under heating conditions). Thereafter, the precipitated red solid was collected by filtration and washed with carbon tetrachloride. The obtained red solid was dried under reduced pressure for 2 hours to obtain a red solid (88 mg) of a DAND radical salt (chloride). The compound was used in a next reaction without further purification. Next, sodium tetrakis[3,5-bis(trifluoromethyl)phenyl]borate hydrate (275 mg, 0.31 mmol) was added at room temperature to a solution of DAND radical salt (chloride) (88 mg) in tetrahydrofuran (3.0 mL). This solution was stirred at room temperature for 2 hours, and then water was added thereto. Organic materials were extracted from the aqueous layer with dichloromethane. The organic layers were dried with sodium sulfate and concentrated under reduced pressure. The obtained residue was purified by silica gel column chromatography to obtain a red solid (100 mg, 87.7 µmol, 30% (2 steps)) of DAND radical salt (BArF salt) in the fraction eluted with chloroform-methanol (20:1 volume ratio).

The results of elemental analysis of the obtained DAND radical salt (BArF salt) are shown below.

Anal. Calcd. for C₄₈H₄₀BF₂₄N₄: for C, 50.59; H, 3.54; N, 4.92; found: C, 50.08; H, 3.66; N, 4.86.

### (Example 11)

### <Synthesis of 1,2-Bis(3-((tertbutyldimethylsilyl)oxy)-9-azabicyclo[3.3.1]nonan-9-yl)diazene>

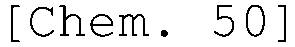

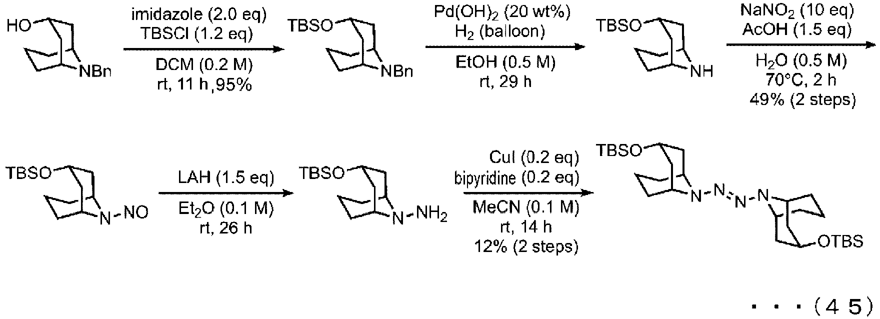

1,2-Bis(3-((tert-butyldimethylsilyl)oxy)-9-azabicyclo[3.3.1]nonan-9-yl)diazene (hereinafter, tertbutyldimethylsilyl was referred to as "TBS", and 1,2-bis(3-((tert-butyldimethylsilyl)oxy)-9-azabicyclo[3.3.1]nonan-9-yl)diazene was referred to as "3-TBS-oxy-DAND") represented by the following chemical formula (46) was synthesized according to a reaction route shown in the above formula (45). A starting material, (3r)-9-benzyl-9-azabicyclo[3.3.1]-3-nonanol, was synthesized based on WO2007/039563.

### <Synthesis of 9-Benzyl-3-TBS-oxy-9-azabicyclo[3.3.1]nonane>

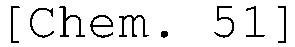

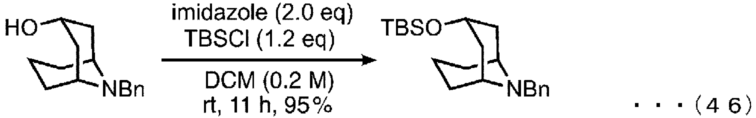

9-Benzyl-3-TBS-oxy-9-azabicyclo[3.3.1]nonane was synthesized according to a reaction route shown in the above formula (46). Specifically, first, imidazole (1.64 g, 24.1 mmol) and TBS chloride (2.18 g, 14.5 mmol) were added at room temperature to a solution of (3r)-9-benzyl-9-azabicyclo[3.3.1]-3-nonanol (2.79 g, 12.1 mmol) in dichloromethane (60 mL), followed by stirring at room temperature for 11 hours. Thereafter, water was added thereto at room temperature, and organic materials were extracted from the aqueous layer with dichloromethane. The organic layers were washed with saturated brine, then dried with magnesium sulfate, and concentrated under reduced pressure. The obtained residue was purified by silica gel column chromatography to obtain a colorless liquid (3.95 g, 11.4 mmol, 95%) of 9-benzyl-3-TBS-oxy-9-azabicyclo[3.3.1]nonane in the fraction eluted with hexane-ethyl acetate (15:1 volume ratio).

The results of ¹H-NMR, ¹³C-NMR, IR, MS and HRMS (EI) of the obtained 9-benzyl-3-TBS-oxy-9-azabicyclo[3.3.1]nonane are shown below.

¹H-NMR (400 MHz): δ 7.30-7.20 (m, 4H), 7.18-7.12 (m, 1H), 4.14 (tt, J = 6.8, 6.8 Hz, 1H), 3.71 (s, 2H), 2.93-2.84 (m, 2H), 2.31 (dtd, J = 18.7, 13.4, 5.1 Hz, 1H), 2.19 (ddd, J = 15.0, 9.1, 6.6 Hz), 1.84 (tt, J = 13.4, 4.9 Hz, 2H), 1.43-1.34 (m, 1H), 1.31 (ddd, J = 13.9, 7.1, 2.5 Hz, 2H), 1.07-1.04 (m, 2H).
¹³C-NMR (100 MHz): δ 140.7, 128.2, 128.1, 126.6, 64.5, 56.1, 49.935.8, 25.9, 25.3, 18.2, 14.8, -4.7.
IR (neat [cm⁻¹]): 2929, 1090, 1032, 835, 775.
MS [m/z]: 345 [M⁺], 91 [100%].
HRMS (EI): Calcd. for C₂₁H₃₅NOSi: 345.2488, found: 345.2419.

### <Synthesis of 3-TBS-Oxy-9-nitroso-9-azabicyclo[3.3.1]nonane>

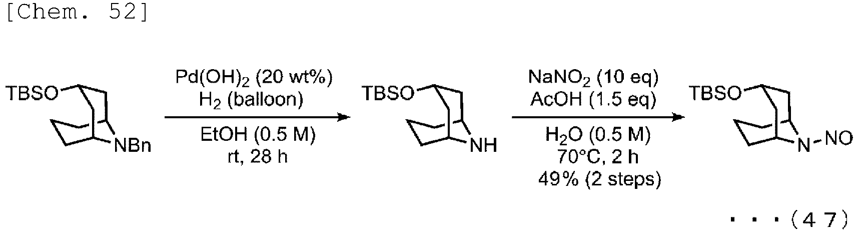

3-TBS-Oxy-9-nitroso-9-azabicyclo[3.3.1]nonane was synthesized according to a reaction route shown in the above formula (47). Specifically, first, palladium(II) hydroxide (Pd 20%, approximately 50% hydrated) (55 mg) was added at room temperature to a solution of 9-benzyl-3-TBS-oxy-9-azabicyclo[3.3.1]nonane (299 mg, 865 µmol) in ethanol (1.7 mL) in a reaction vessel purged with argon gas. The vessel was filled with hydrogen gas (1 atm), followed by stirring at room temperature for 28 hours. Thereafter, the reaction vessel was purged with argon gas, and the reaction solution was subjected to Celite filtration. The obtained filtrate was concentrated under reduced pressure. The obtained residue was diluted with chloroform, dried with potassium carbonate, and concentrated under reduced pressure to obtain a pale yellow oil of 3-TBS-oxy-9-azabicyclo[3.3.1]nonane (174 mg). The compound was used in a next reaction without further purification.

Next, acetic acid (58.3 µL, 1.02 mmol) and sodium nitrite (469 mg, 6.79 mmol) were added at room temperature to a solution of the obtained pale yellow oil (174 mg) in water (1.4 mL). This solution was stirred at 70 °C for 2 hours, and then cooled to room temperature, and organic materials were extracted from the aqueous layer with diethyl ether. The organic layers were washed with saturated brine, then dried with sodium sulfate, and concentrated under reduced pressure. The obtained residue was purified by silica gel column chromatography to obtain a yellow solid of 3-TBS-oxy-9-nitroso-9-azabicyclo[3.3.1]nonane (121 mg, 49% (2 steps)) in the fraction eluted with hexane-ethyl acetate (5:1 volume ratio).

The results of ¹H-NMR, ¹³C-NMR, IR, MS, and HRMS (EI) of the obtained 3-TBS-oxy-9-nitroso-9-azabicyclo[3.3.1]nonane are shown below.

¹H-NMR (400 MHz): δ 5.34 (d, J = 5.5 Hz, 1H), 4.95 (d, J = 6.3 Hz, 1H), 3.59-3.51 (m, 1H), 2.50-2.32 (m, 2H), 2.09 (dddd, J = 13.7, 9.9, 6.3, 1.9 Hz, 1H), 1.85-1.46 (m, 7H), 0.87 (d, J = 2.2 Hz, 9H), 0.02 (d, J = 2.9 Hz, 6H).
¹³C-NMR (100 MHz): δ 63.6, 52.9, 41.7, 37.0.35.2, 32.2, 29.9, 25.7, 17.9, 14.6, -4.9.
IR (neat [cm⁻¹]): 2931, 2856, 1435, 1367, 1103.
MS [m/z]: 284 [M⁺], 227 [100%].
HRMS (EI): Calcd. for C₁₄H₂₈N₂O₂Si: 284.1920, found: 284.1913.

### <Synthesis of 3-TBS-Oxy-DAND>

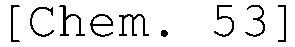

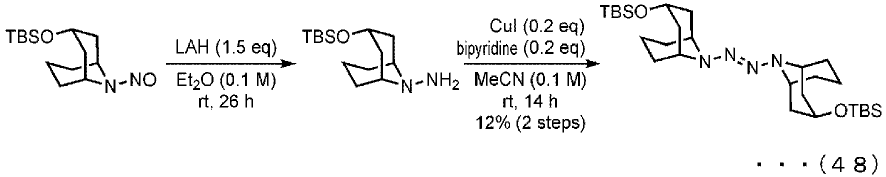

3-TBS-Oxy-DAND was synthesized according to a reaction route shown in the above formula (48). Specifically, first, a solution of 3-TBS-oxy-9-nitroso-9-azabicyclo[3.3.1]nonane (121 mg, 425 µmol) in diethyl ether (Et₂O, 708 µL) was slowly added at 0°C to a solution of LAH (24 mg, 637 umol) in diethyl ether (708 µL). This solution was stirred at room temperature for 3 hours, then diethyl ether (1.4 mL) was added thereto and stirred for 5.5 hours, and then diethyl ether (2.8 mL) was further added thereto and stirred for 17.5 hours. Thereafter, diethyl ether, water, and a 10% sodium hydroxide solution were each slowly added at 0°C, followed by stirring at room temperature for 15 minutes. Thereafter, Celite filtration was performed, and concentration was performed under reduced pressure to obtain a white solid (94 mg) containing 3-TBS-oxy-9-azabicyclo[3.3.1]nonan-9-amine. The compound was used in a next reaction without further purification. Next, copper iodide (12.3 mg, 64.4 µmol) and 2,2'-bipyridine (10.6 mg, 64.4 µmol) were dissolved in acetonitrile (1.5 mL). To the solution, a solution of the previously synthesized white solid (87 mg) containing 3-TBS-oxy-9-azabicyclo[3.3.1]nonan-9-amine in acetonitrile (1.8 mL) was slowly added dropwise over 3 hours. Thereafter, the mixture was stirred in open air at room temperature for 14 hours, and then tetramethylethylenediamine (19 µL, 129 µmol) was added thereto, followed by stirring for 10 minutes. Water was added to the solution, and organic materials were extracted from the aqueous layer with dichloromethane. The organic layers were further washed with saturated brine, then dried with sodium sulfate, and concentrated under reduced pressure. The obtained residue was purified by silica gel column chromatography to obtain a white solid of 3-TBS-oxy-DAND (13.6 mg, 25.2 µmol, yield 12% (2 steps)) in the fraction eluted with hexane-ethyl acetate (30:1 volume ratio to 15:1 volume ratio).

The results of ¹H-NMR, ¹³C-NMR, IR, MS, and HRMS (EI) of the obtained 3-TBS-oxy-DAND are shown below.

¹H-NMR (400 MHz): δ 4.25-4.17 (m, 4H), 3.91-3.80 (m, 2H), 2.36-2.17 (m, 6H), 1.75 (t, J = 23 Hz, 4H), 1.56-1.35 (m, 10H), 0.88 (s, 18H), 0.03 (s, 12H).
¹³C-NMR (100 MHz): δ -4.8, 0.0, 14.8, 18.1, 25.9, 29.0, 35.2, 49.2, 64.2.
IR (neat [cm⁻¹]): 3394, 2931, 1066, 773.
MS [m/z]: 536 [M⁺, 100%].
HRMS (EI): Calcd. for C₂₈H₅₆N₄O₂Si₂: 536.3942, found: 536.3943.

### (Example 12)

### <Synthesis of 1,2-Di(8-azabicyclo[3.2.1]octan-8-yl)diazene>

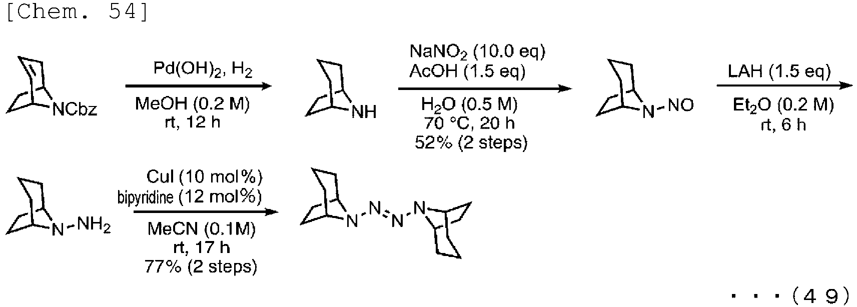

1,2-Di(8-azabicyclo[3.2.1]octan-8-yl)diazene (hereinafter referred to as "DAOD") represented by the following chemical formula (50) was synthesized according to a reaction route shown in the above formula (49). A starting material, benzyl 8-azabicyclo[3.2.1]octene-8-carboxylate, was synthesized using a method reported by Toda et al. (Toda, M. et al., J. Org. Chem., 2023, Vol. 88, No. 3, pp. 1434-1444).

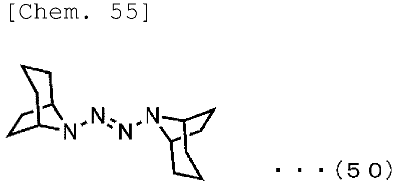

### <Synthesis of 8-Nitroso-8-azabicyclo[3.2.1]octane>

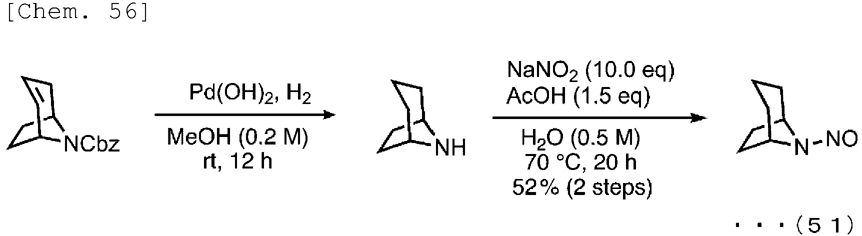

8-Nitroso-8-azabicyclo[3.2.1]octane was synthesized according to a reaction route shown in the above formula (51). Specifically, first, palladium hydroxide on activated carbon (Pd 20%, approximately 50% hydrated) (143 mg) was added at room temperature to a solution of benzyl 8-azabicyclo[3.2.1]octene-8-carboxylate (1.43 g, 5.88 mmol) in acetonitrile (29 mL) in a reaction vessel purged with argon gas. The reaction vessel was filled with hydrogen gas (1 atm), followed by stirring at room temperature for 12 hours. Thereafter, the reaction vessel was purged with argon gas, and the reaction solution was subjected to Celite filtration. The obtained filtrate was concentrated under reduced pressure to obtain a white solid of 8-azabicyclo[3.2.1]octane (697 mg). The compound was used in a next reaction without further purification. Next, acetic acid (0.56 mL, 9.4 mmol) and sodium nitrite (4.32 g, 62.7 mmol) were added at room temperature to a solution of the obtained white solid (697 mg) in water (13 mL). This solution was stirred at 70 °C for 20 hours, and then cooled to room temperature, and organic materials were extracted from the aqueous layer with diethyl ether. The organic layers were washed with saturated brine, then dried with sodium sulfate, and concentrated under reduced pressure. The obtained residue was purified by silica gel column chromatography to obtain a yellow solid of 8-nitroso-8-azabicyclo[3.2.1]octane (479 mg, 3.07 mmol, 58% (2 steps)) in the fraction eluted with hexane-ethyl acetate (5:1 volume ratio).

The results of ¹H-NMR, ¹³C-NMR, IR, MS, and HRMS (EI) of the obtained 8-nitroso-8-azabicyclo[3.2.1]octane are shown below.

¹H-NMR (400 MHz): δ 5.13-5.06 (m, 1H), 4.93 (d, J = 7.2 Hz, 1H), 2.24-2.10 (m, 1H), 2.00-1.45 (m, 9H).
¹³C-NMR (100 MHz): δ 58.4, 50.6, 34.2, 30.4, 26.1, 25.5, 16.3.
IR (neat [cm⁻¹]): 2942, 2880, 1476, 1405, 1347, 1280, 730.
MS [m/z]: 140 (M⁺), 110 (100%).
HRMS (EI): Calcd. for C₇H₁₂N₂O: 140.1860, found: 140.0956.

### <Synthesis of DAOD>

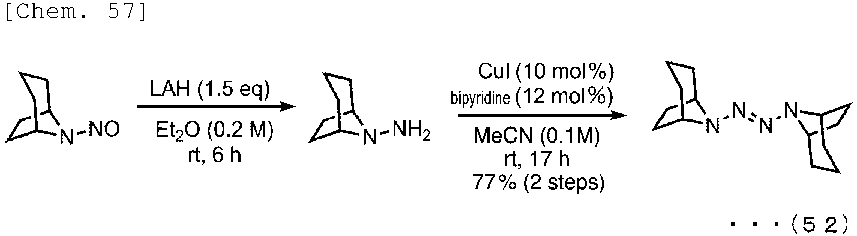

DAOD was synthesized according to a reaction route shown in the above formula (52). Specifically, first, a solution of 8-nitroso-8-azabicyclo[3.2.1]octane (300 mg, 1.92 mmol) in diethyl ether (Et₂O, 6.0 mL) was slowly added at 0 °C to a solution of LAH (109 mg, 2.88 mmol) in diethyl ether (13 mL). This solution was stirred at room temperature for 1 hour, and then diethyl ether, water, and a 10% sodium hydroxide solution were each slowly added thereto at 0 °C, followed by stirring at room temperature for 30 minutes. Thereafter, Celite filtration was performed, and concentration was performed under reduced pressure to obtain a white solid of 8-azabicyclo[3.2.1]octan-8-amine (262 mg). The product was used in a next reaction without further purification. Next, copper iodide (39.5 mg, 208 µmol) and 2,2'-bipyridine (38.9 mg, 249 µmol) were dissolved in acetonitrile (8 mL). To the solution, a solution of the previously synthesized 8-azabicyclo[3.2.1]octan-8-amine (262 mg) in acetonitrile (12 mL) was slowly added dropwise at room temperature over 7 hours. Thereafter, the mixture was stirred in open air at room temperature for 17 hours, and then tetramethylethylenediamine (62 µL, 415 µmol) was added thereto, followed by stirring for 10 minutes. Water was added to the solution, and organic materials were extracted from the aqueous layer with dichloromethane. The organic layers were further washed with saturated brine, then dried with sodium sulfate, and concentrated under reduced pressure. The obtained residue was purified by silica gel column chromatography to obtain a white solid of DAOD (183 mg, 737 µmol, yield 77% (2 steps)) in the fraction eluted with hexane-ethyl acetate (15:1 volume ratio).

The results of ¹H-NMR, ¹³C-NMR, IR, MS, and HRMS (EI) of the obtained DAOD are shown below.

¹H-NMR (400 MHz): δ 4.16 (s, 4H), 1.91-1.37 (m, 20H).
¹³C-NMR (100 MHz): δ 58.1, 57.9, 29.7, 26.4, 16.9.
IR (neat [cm⁻¹]): 2942, 2868, 1445, 1335, 1231, 1016, 967, 951, 755.
MS [m/z]: 248 (M⁺), 110 (100%).
HRMS (EI): Calcd. for C₁₄H₂₄N₄: 248.2001, found: 248.2007.

### <Activity Evaluation 1>

Using the DAD obtained in Example 1 as a catalyst, an oxidation reaction of an alcohol was performed according to a reaction route shown in the following formula (53).

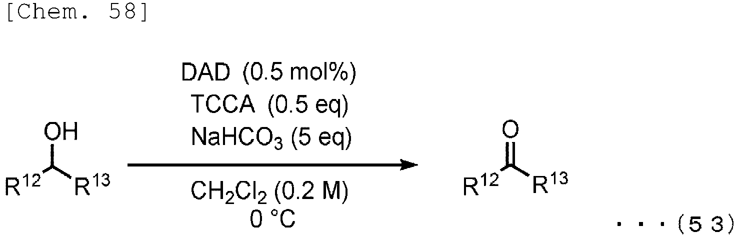

An oxidation reaction of various alcohols was performed according to the reaction route shown in the above formula (53). Specifically, an alcohol (substrate, 1.00 mmol) shown in Table 1, DAD (1.50 mg, 5 µmol), and sodium hydrogen carbonate (NaHCO₃, 420 mg, 5.00 mmol) were dissolved or suspended in dichloromethane (CH₂Cl₂, 5.0 mL) and stirred under ice cooling. Subsequently, trichloroisocyanuric acid (the following chemical formula (54), hereinafter referred to as TCCA, 116 mg, 0.500 mmol) was added to the reaction solution, followed by stirring at the same temperature. After confirming the completion of the reaction, a 20% aqueous sodium thiosulfate solution was added to the reaction solution, followed by extracting with dichloromethane. The obtained organic layers were dried with sodium sulfate and then concentrated under reduced pressure. The residue was purified by silica gel column chromatography to obtain a target compound (product, yield 74% to 94%).

In Table 1, a indicates that 5 mol% of DAD was added to the alcohol, and b indicates an NMR yield. Ph represents a phenyl group, TBS represents a tert-butyldimethylsilyl group, and Cbz represents a benzyloxycarbonyl group.

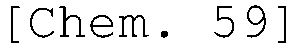

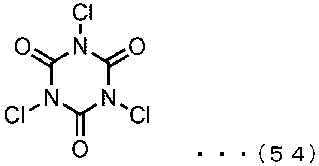

**[Table 1]**

| 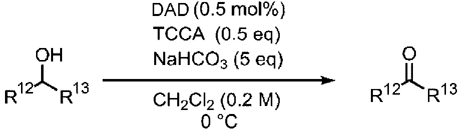 | | | | |
|---|---|---|---|---|
| entry | substrate | product | time (h) | yield (%) |
| 1 | 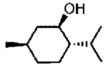 | 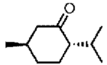 | 4 | 86 |
| 2 | 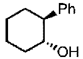 | 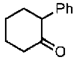 | 4.5 | 87 |
| 3 | 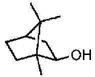 | 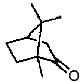 | 3 | 86 |
| 4 | 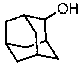 | 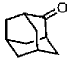 | 2 | 88 |
| 5 | 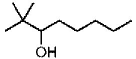 | 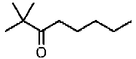 | 4.5 | 89 |
| 6 | 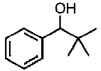 | 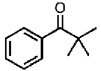 | 12 | 94 |
| 7 | 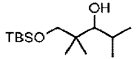 | 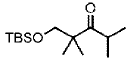 | 0.5 | 93 |
| 8*^{a}* | 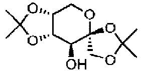 | 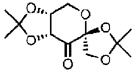 | 6 | 80 |
| 9*^{a}* | 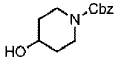 | 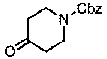 | 4 | 89 |
| 10 | 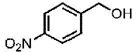 | 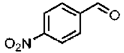 | 7 | 84 |
| 11*^{b}* | 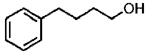 | 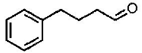 | 4 | 74 |

As is clear from the results shown in Table 1, it was confirmed that the alcohol oxidation catalyst containing DAD of the invention functions as an oxidation catalyst for various alcohols, and an aldehyde or a ketone corresponding to each alcohol can be obtained as a target compound at a high yield. It was confirmed that the target compound was obtained at a high yield even in an alcohol having a three-dimensionally bulky and complicated structure, and not only simple benzyl alcohols and aliphatic alcohols but also a wide range of alcohols such as heteroatom-containing alcohols and sugar alcohols could be oxidized as a substrate.

### <Activity Evaluation 2>

Using the DAD obtained in Example 1 as a catalyst, an oxidation reaction of an alcohol was performed according to a reaction route shown in the following formula (55).

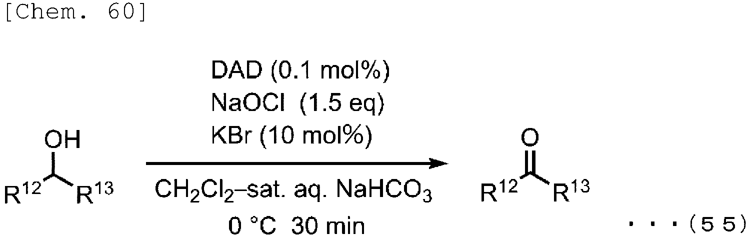

An oxidation reaction of various alcohols was performed according to the reaction route shown in the above formula (55). Specifically, an alcohol (substrate, 1.00 mmol) shown in Table 2, DAD (300 µg, 1.00 µmol), and potassium bromide (KBr, 11.9 mg, 0.100 mmol) were dissolved in dichloromethane (CH₂Cl₂, 2.5 mL), and 1.25 mL of saturated sodium bicarbonate solution was further added thereto, followed by stirring under ice cooling. Next, a mixed solution of 1.54 mol/L sodium hypochlorite (1.0 mL, 1.5 mmol) and saturated sodium bicarbonate solution (1.25 mL) was slowly added to the reaction solution, followed by stirring at the same temperature. Thereafter, isopropanol was added to the reaction solution, followed by stirring for 10 minutes. The reaction solution was extracted with dichloromethane, and the obtained organic layers were washed with saturated brine. The obtained organic layers were dried with sodium sulfate and then concentrated under reduced pressure. The obtained residue was purified by silica gel column chromatography to obtain a target compound (product, yield 85% to 98%).

In Table 2, a indicates that a reaction time was 1 hour, and b indicates that 0.5 mol% of DAD was added to the alcohol. c indicates that 1.2 equivalents of sodium hypochlorite was used. Bz represents a benzoyl group, TBS represents a tert-butyldimethylsilyl group, and Cbz represents a benzyloxycarbonyl group.

**[Table 2]**

| 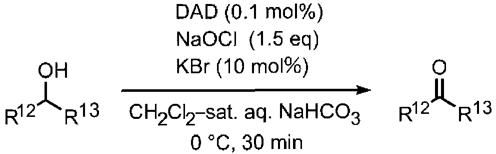 | | | |
|---|---|---|---|
| entry | substrate | product | yield (%) |
| 1 | 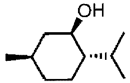 | 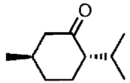 | 96 |
| 2 | 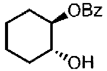 | 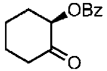 | 98 |
| 3 | 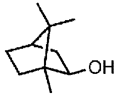 | 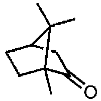 | 85 |
| 4^{a} | 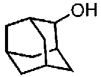 | 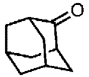 | 85 |
| 5 | 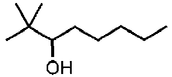 | 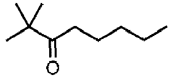 | 92 |
| 6^{a,b} | 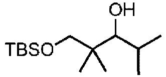 | 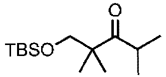 | 97 |
| 7 | 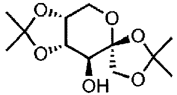 | 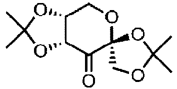 | 98 |
| 8 | 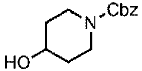 | 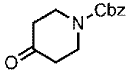 | 98 |
| 9*^{a,c}* | 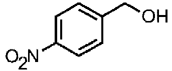 | 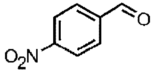 | 89 |
| 10 | 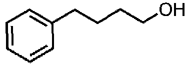 | 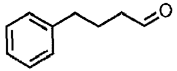 | 90 |

### <Activity Evaluation 3>

Using the DAD radical salt (BArF salt) obtained in Example 2 as a catalyst, an oxidation reaction of menthol was performed according to a reaction route shown in the following formula (56).

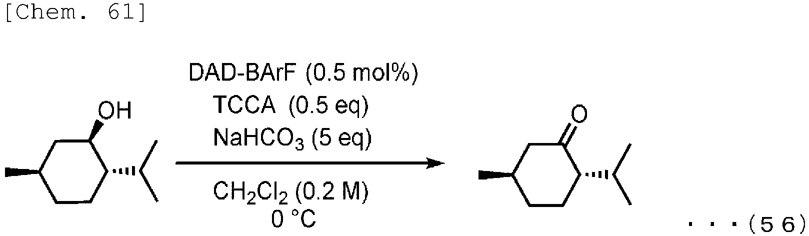

Menthol (40 mg, 0.26 mmol), DAD radical salt (BArF salt or DAD-BArF) (1.5 mg, 1.3 µmol), and sodium hydrogen carbonate (NaHCO₃, 110 mg, 1.30 mmol) were dissolved or suspended in dichloromethane (CH₂Cl₂, 1.3 mL) and stirred under ice cooling. Thereafter, TCCA (the above chemical formula (54), 30 mg, 0.13 mmol) was added to the reaction solution, followed by reacting at the same temperature for 6 hours. Next, 10 µL of the reaction solution after the reaction was taken and diluted with 0.5 mL of isopropanol to prepare a sample, and the sample was analyzed using gas chromatography. As a result, six hours after the start of the reaction, a conversion rate reached 89%.

As is clear from the above results, it was confirmed that the alcohol oxidation catalyst containing the DAD radical salt (BArF salt) of the invention functions as an oxidation catalyst for an alcohol, and a ketone corresponding to the alcohol as a target compound can be obtained at a high yield.

### <Activity Evaluation 4>

Using the DAD obtained in Example 1 as a catalyst, an oxidation reaction of an alcohol was performed according to a reaction route shown in the following formula (57).

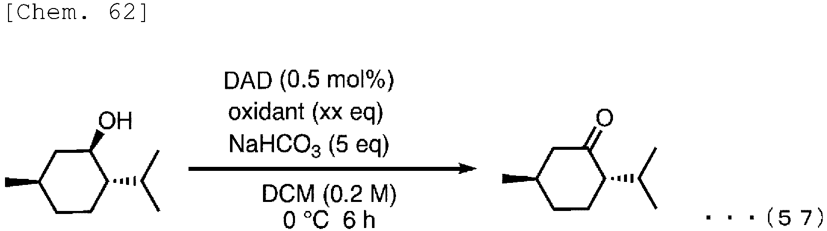

In the case of using 1,3-dichloro-5,5-dimethylhydantoin (101 mg, 0.512 mmol) represented by the following chemical formula (58) instead of TCCA, an oxidation reaction of menthol that is represented by the above formula (57) was performed as in Activity Evaluation 1. A stirring time under ice cooling was 6 hours.

The reaction was evaluated by gas chromatography to confirm the generation of the target compound (conversion rate 33%).

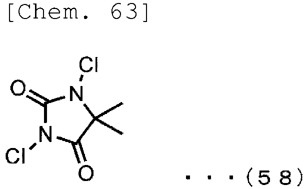

### <Activity Evaluation 5>

An oxidation reaction of menthol that is represented by the above formula (57) was performed as in Activity Evaluation 1 except that 1,3-dibromo-5,5-dimethylhydantoin (146 mg, 0.512 mmol) represented by the following chemical formula (59) was used instead of TCCA. A stirring (reaction) time under ice cooling was 6 hours.

The reaction was evaluated by gas chromatography to confirm the generation of the target compound (conversion rate 43%).

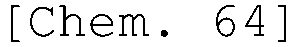

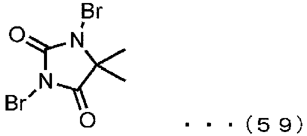

### <Activity Evaluation 6>

An oxidation reaction of menthol that is represented by the above formula (57) was performed as in Activity Evaluation 1 except that N-chlorosaccharin (245 mg, 1.13 mmol) represented by the following chemical formula (60) was used instead of TCCA. A stirring (reaction) time under ice cooling was 6 hours.

The reaction was evaluated by gas chromatography to confirm the generation of the target compound (conversion rate 39%).

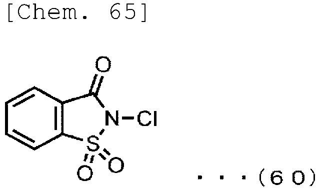

As is clear from the above results, it was confirmed that the alcohol oxidation catalyst containing DAD of the invention functions as an oxidation catalyst for an alcohol even when a cooxidant is changed, and an aldehyde or a ketone corresponding to each alcohol can be obtained as a target compound at a high yield.

### <Activity Evaluation 7>

### <Comparison of Alcohol Oxidation Catalytic Activity Based on Differences in Main Skeleton and Substituent>

An oxidation reaction of an alcohol was performed using sodium hypochlorite as an oxidant and menthol as a substrate, as in a reaction route shown in the following formula (61). Specifically, menthol (156 mg, 1.00 mmol), a catalyst (catalyst) (0.1 mol%) shown in Table 3, and potassium bromide (KBr, 11 mg, 10.0 mol%) were dissolved in a mixed solution of dichloromethane (CH₂Cl₂, 2.5 mL) and a saturated aqueous sodium bicarbonate solution (1.25 mL), followed by stirring under ice cooling. A mixed solution of 1.54 mol/L aqueous sodium hypochlorite solution (1.0 mL, 1.5 mmol) and a saturated aqueous sodium bicarbonate solution (1.25 mL) was slowly added dropwise to the reaction solution and stirred at the same temperature. Next, 10 µL of the reaction solution (dichloromethane layer) was taken and diluted with 0.5 mL of isopropanol to prepare a sample, and a conversion rate of the sample was evaluated by gas chromatography 30 minutes after the start of the reaction.

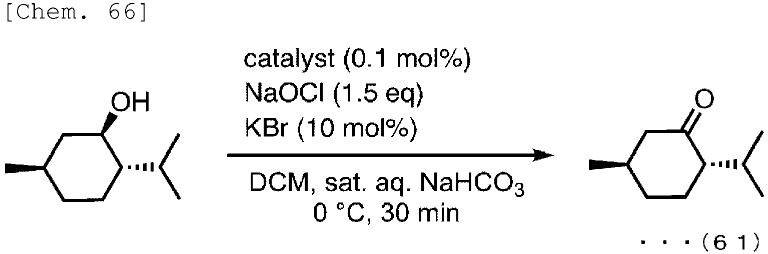

**[Table 3]**

| 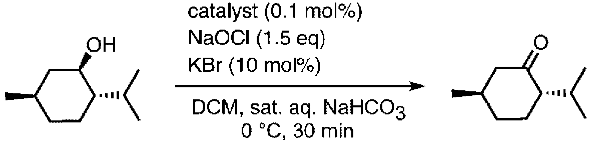 | | | |
|---|---|---|---|
| catalyst | GC conv. | catalyst | GC conv. |
| 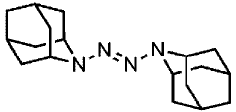 | 100% | 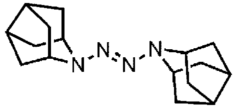 | 100% |
| 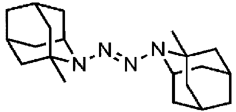 | 99% | 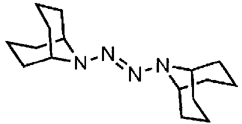 | 100% |
| 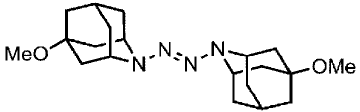 | 98% | 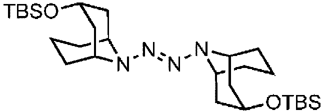 | 100% |
| 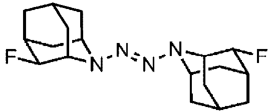 | 95% | 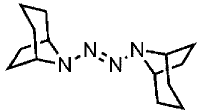 | 94 % |

As is clear from the above results, it was confirmed that the tetrazene-type alcohol oxidation catalysts of the invention function as oxidation catalysts for an alcohol even when a main skeletons and a substituents are changed, and an aldehyde or a ketone corresponding to each alcohol can be obtained as a target compound at a high yield.

### <Comparison of Catalyst Current by Cyclic Voltammetry (CV)>

Using cyclic voltammetry (CV), catalyst currents of the DAD obtained in Example 1 and AZADO were compared.

Measurement conditions of the cyclic voltammetry were as follows.

Tetrabutylammonium hexafluorophosphate (TBAPF₆, 387 mg, 1.00 mmol), DAD or AZADO (10 µmol) was dissolved in acetonitrile (CH₃CN, 10 mL). Next, a measurement was performed at a sweep rate of 10 mV/s using glassy carbon as an anode, a platinum wire as a cathode, and an Ag/AgNO₃ electrode as a reference electrode. Thereafter, menthol (156 mg, 1.00 mmol) and 2,4,6-collidine (66 µL, 0.50 mmol) were added, and the measurement was performed again under the same conditions.

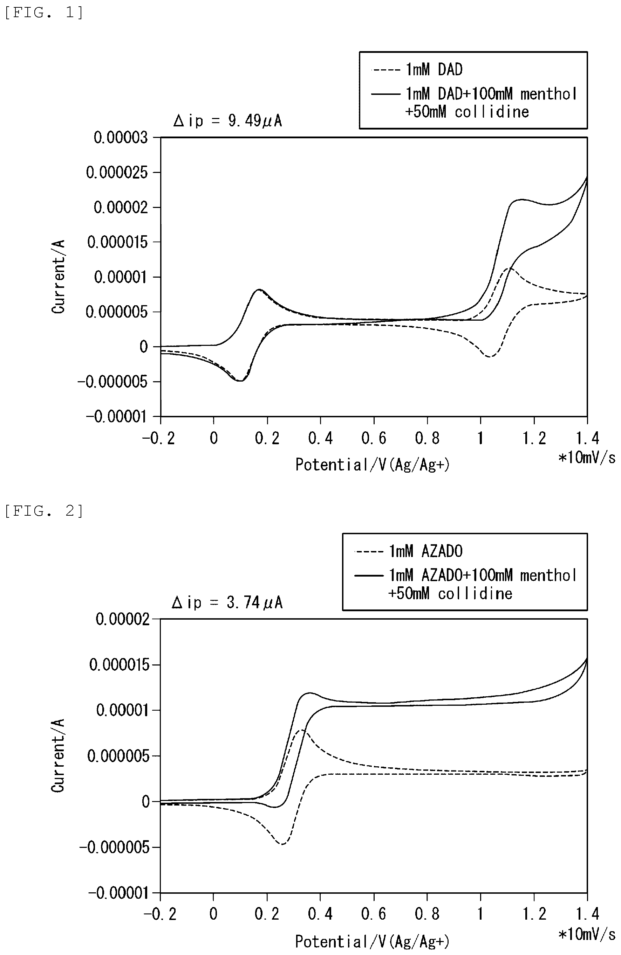

The results are shown in FIGS. 1 and 2.

FIG. 1 is a diagram illustrating a measurement result of the cyclic voltammetry of DAD. In FIG. 1, a broken line indicates the cyclic voltammetry of DAD, and a solid line indicates the cyclic voltammetry when DAD is used as an oxidation catalyst for menthol. FIG. 2 is a diagram illustrating a measurement result of the cyclic voltammetry of AZADO. In FIG. 2, a broken line indicates the cyclic voltammetry of AZADO, and a solid line indicates the cyclic voltammetry when AZADO is used as an oxidation catalyst for menthol.

From the results shown in FIG. 1, it was found that a difference (Δip) between an oxidation current in the cyclic voltammetry of DAD and an oxidation current in the cyclic voltammetry when DAD was used as an oxidation catalyst for menthol was 9.49 µA. On the other hand, from the results shown in FIG. 2, it was found that a difference (Δip) between an oxidation current in the cyclic voltammetry of AZADO and an oxidation current in the cyclic voltammetry when AZADO was used as an oxidation catalyst for menthol was 3.74 µA. From the above results, it was found that when menthol was used as the alcohol, DAD exhibited a catalyst current larger than that of AZADO. This suggests that a rate of the oxidation reaction of menthol by DAD is higher than a rate of the oxidation reaction of menthol by AZADO.

### Industrial Applicability

As described above, according to the invention, new tetrazene compounds, new tetrazene radical salt compounds, new tetrazene-type alcohol oxidation catalysts, and new tetrazene radical salt-type alcohol oxidation catalysts that are each suitably applicable as alcohol oxidation catalysts capable of exhibiting a sufficiently high catalytic activity for primary and secondary alcohols and that are easy to produce, and alcohol oxidation methods using them can be provided. Therefore, the invention is highly useful because it can be applied to a catalytic oxidation of alcohols, which serves as an environmentally benign method for synthesizing fine chemicals such as pharmaceuticals, pharmaceutical intermediates, agrochemicals, cosmetics, and organic materials.

## Claims

1. A tetrazene compound having an adamantane skeleton represented by the following chemical formula (1) or a bicyclic skeleton represented by the following chemical formula (2): 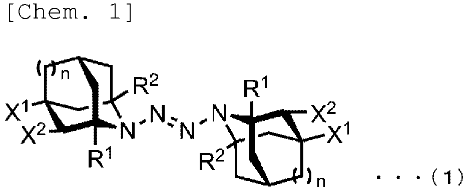 (in the formula (1), n is 0 or 1, R¹ is H, an alkyl group having 1 to 6 carbon atoms that may include a substituent, a phenyl group that may include a substituent, a carboxy group, an amide group, or an alkoxycarbonyl group having 1 to 6 carbon atoms, R² is H, an alkyl group having 1 to 6 carbon atoms that may include a substituent, a carboxy group, an amide group, or an alkoxycarbonyl group having 1 to 6 carbon atoms, X¹ is H, OR³ (R³ is H, an alkyl group having 1 to 6 carbon atoms that may include a substituent, an acyl group, or a silyl group having 3 to 18 carbon atoms), NR⁴R⁵ (R⁴ and R⁵ are each independently H, an alkyl group having 1 to 6 carbon atoms that may include a substituent, an acyl group, an alkoxycarbonyl group, or a group obtained by substituting OH of a sulfo group with a monovalent group, and R⁴ and R⁵ may be bonded to each other to form a ring), a halogen atom, a phenyl group that may include a substituent, or an alkyl group having 1 to 6 carbon atoms that may include a substituent, X² is H, OR³ (R³ is H, an alkyl group having 1 to 6 carbon atoms that may include a substituent, an acyl group, or a silyl group having 3 to 18 carbon atoms), NR⁴R⁵ (R⁴ and R⁵ are each independently H, an alkyl group having 1 to 6 carbon atoms that may include a substituent, an acyl group, an alkoxycarbonyl group, or a group obtained by substituting OH of a sulfo group with a monovalent group, and R⁴ and R⁵ may be bonded to each other to form a ring), or a halogen atom, and either X¹ or X² is H) 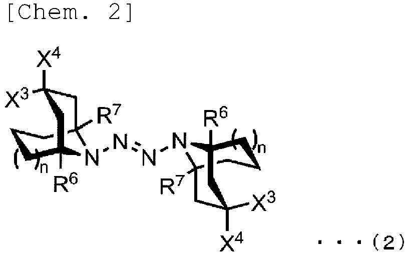 (in the formula (2), n is 0 or 1, R⁶ and R⁷ are each independently H or an alkyl group having 1 to 6 carbon atoms that may include a substituent, X³ and X⁴ are each independently H, an alkyl group having 1 to 6 carbon atoms that may include a substituent, an aromatic group that may include a substituent, OR³ (R³ is H, an alkyl group having 1 to 6 carbon atoms that may include a substituent, an acyl group, or a silyl group having 3 to 18 carbon atoms), NR⁴R⁵ (R⁴ and R⁵ are each independently H, an alkyl group having 1 to 6 carbon atoms that may include a substituent, an acyl group, an alkoxycarbonyl group, or a group obtained by substituting OH of a sulfo group with a monovalent group, and R⁴ and R⁵ may be bonded to each other to form a ring), or a halogen atom, or X³ and X⁴ may form a keto group, an imino group (NR⁸), an oxime group (NOR⁹), or a hydrazone group (NNR¹⁰R¹¹), the R⁸ to R¹¹ are each independently H, an alkyl group having 1 to 6 carbon atoms that may include a substituent, a phenyl group that may include a substituent, an acyl group, an alkoxycarbonyl group, or a group obtained by substituting OH of a sulfo group with a monovalent group, R¹⁰ and R¹¹ may be bonded to each other to form a ring, X³ and X⁴ may be bonded to each other to form a ring, and one of R⁶, R⁷, X³, and X⁴ is not H).

2. A tetrazene radical salt compound having an adamantane skeleton represented by the following chemical formula (3) or a bicyclic skeleton represented by the following chemical formula (4): 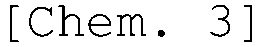 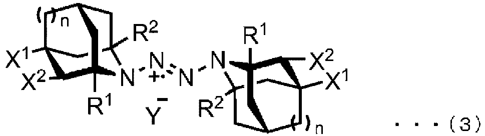 (in the formula (3), n is 0 or 1, R¹ is H, an alkyl group having 1 to 6 carbon atoms that may include a substituent, a phenyl group that may include a substituent, a carboxy group, an amide group, or an alkoxycarbonyl group having 1 to 6 carbon atoms, R² is H, an alkyl group having 1 to 6 carbon atoms that may include a substituent, a carboxy group, an amide group, or an alkoxycarbonyl group having 1 to 6 carbon atoms, X¹ is H, OR³ (R³ is H, an alkyl group having 1 to 6 carbon atoms that may include a substituent, an acyl group, or a silyl group having 3 to 18 carbon atoms), NR⁴R⁵ (R⁴ and R⁵ are each independently H, an alkyl group having 1 to 6 carbon atoms that may include a substituent, an acyl group, an alkoxycarbonyl group, or a group obtained by substituting OH of a sulfo group with a monovalent group, and R⁴ and R⁵ may be bonded to each other to form a ring), a halogen atom, a phenyl group that may include a substituent, or an alkyl group having 1 to 6 carbon atoms that may include a substituent, X² is H, OR³ (R³ is H, an alkyl group having 1 to 6 carbon atoms that may include a substituent, an acyl group, or a silyl group having 3 to 18 carbon atoms), NR⁴R⁵ (R⁴ and R⁵ are each independently H, an alkyl group having 1 to 6 carbon atoms that may include a substituent, an acyl group, an alkoxycarbonyl group, or a group obtained by substituting OH of a sulfo group with a monovalent group, and R⁴ and R⁵ may be bonded to each other to form a ring), or a halogen atom, either X¹ or X² is H, and Y⁻ is a monovalent anion) 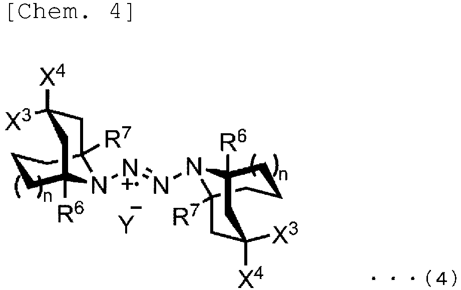 (in the formula (4), n is 0 or 1, R⁶ and R⁷ are each independently H or an alkyl group having 1 to 6 carbon atoms that may include a substituent, X³ and X⁴ are each independently H, an alkyl group having 1 to 6 carbon atoms that may include a substituent, an aromatic group that may include a substituent, OR³ (R³ is H, an alkyl group having 1 to 6 carbon atoms that may include a substituent, an acyl group, or a silyl group having 3 to 18 carbon atoms), NR⁴R⁵ (R⁴ and R⁵ are each independently H, an alkyl group having 1 to 6 carbon atoms that may include a substituent, an acyl group, an alkoxycarbonyl group, or a group obtained by substituting OH of a sulfo group with a monovalent group, and R⁴ and R⁵ may be bonded to each other to form a ring), or a halogen atom, or X³ and X⁴ may form a keto group, an imino group (NR⁸), an oxime group (NOR⁹), or a hydrazone group (NNR¹⁰R¹¹), the R⁸ to R¹¹ are each independently H, an alkyl group having 1 to 6 carbon atoms that may include a substituent, a phenyl group that may include a substituent, an acyl group, an alkoxycarbonyl group, or a group obtained by substituting OH of a sulfo group with a monovalent group, R¹⁰ and R¹¹ may be bonded to each other to form a ring, X³ and X⁴ may be bonded to each other to form a ring, and Y⁻ is a monovalent anion).

3. The tetrazene radical salt compound according to claim 2, wherein
Y⁻ in the chemical formula (3) or the chemical formula (4) is a conjugate base of a strong acid or a conjugate base of a weak acid.

4. A tetrazene-type alcohol oxidation catalyst which is an alcohol oxidation catalyst for oxidizing an alcohol, the catalyst comprising:
at least one selected from the group consisting of the tetrazene compound according to claim 1 and a derivative thereof.

5. A tetrazene radical salt-type alcohol oxidation catalyst which is an alcohol oxidation catalyst for oxidizing an alcohol, the catalyst comprising:
at least one selected from the group consisting of the tetrazene radical salt compound according to claim 2 and a derivative thereof.

6. A tetrazene-type alcohol oxidation catalyst which is an alcohol oxidation catalyst for oxidizing an alcohol, the catalyst comprising:
a tetrazene compound having a bicyclic skeleton represented by the following chemical formula (5): 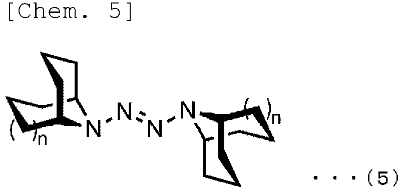
(in the formula, n is 0 or 1).

7. An alcohol oxidation method comprising:
oxidizing an alcohol in presence of the tetrazene-type alcohol oxidation catalyst according to claim 4 and a cooxidant.

8. The alcohol oxidation method according to claim 7, wherein
the alcohol is a primary alcohol or a secondary alcohol.

9. The alcohol oxidation method according to claim 7 or 8, wherein
an addition amount of the tetrazene-type alcohol oxidation catalyst is 0.01 mol or more and 100 mol or less with respect to 100 mol of all hydroxyl groups in the alcohol.

10. An alcohol oxidation method comprising:
oxidizing an alcohol in presence of the tetrazene radical salt-type alcohol oxidation catalyst according to claim 5 and a cooxidant.

11. The alcohol oxidation method according to claim 10, wherein
the alcohol is a primary alcohol or a secondary alcohol.

12. The alcohol oxidation method according to claim 10 or 11, wherein
an addition amount of the tetrazene radical salt-type alcohol oxidation catalyst is 0.01 mol or more and 100 mol or less with respect to 100 mol of all hydroxyl groups in the alcohol.

13. An alcohol oxidation method comprising:
oxidizing an alcohol in presence of the tetrazene-type alcohol oxidation catalyst according to claim 6 and a cooxidant.

14. The alcohol oxidation method according to claim 13, wherein
the alcohol is a primary alcohol or a secondary alcohol.

15. The alcohol oxidation method according to claim 13 or 14, wherein
an addition amount of the tetrazene-type alcohol oxidation catalyst is 0.01 mol or more and 100 mol or less with respect to 100 mol of all hydroxyl groups in the alcohol.
